# EUROPEAN PATENT APPLICATION

(11) **EP 4 825 344 A1**
(43) Date of publication of application: **21.10.2026**
(21) Application number: 25899734.5
(22) Date of filing: 28.04.2025
(51) Int. Cl.: A61F 2/06

(54) **LIMITING EXPANDABLE AORTIC VALVULAR CONDUIT AND PREPARATION THEREFOR**

(30) Priority: 06.12.2024 CN 202411794997
(71) Applicant: Beijing Balance Medical Technology Co., Ltd., Beijing 102200 (CN); Tianjin Chest Hospital, Tianjin 300051 (CN)
(72) Inventor: JIN, Lei, Beijing 102200 (CN); ZHU, Junming, Beijing 100029 (CN); JIANG, Nan, Tianjin 300051 (CN); ZHANG, Xiaoxue, Beijing 102200 (CN); FAN, Zhihao, Beijing 102200 (CN); LU, Hong, Beijing 102200 (CN); HAN, Caili, Beijing 102200 (CN); JIN, Can, Beijing 102200 (CN); WU, Jia, Beijing 102200 (CN); WU, Kangjian, Beijing 102200 (CN); QU, Xiaohuan, Beijing 102200 (CN)
(74) Representative: Patentanwälte und Rechtsanwalt Weiß, Arat & Partner mbB
(86) International application number: PCT/CN2025/091770
(87) International publication number: WO 2026/118364

(57) **Abstract**

The present invention discloses a limiting expandable aortic valve conduit which comprises an aortic valve preliminary conduit attached with a collagen fiber coating, wherein the aortic valve preliminary conduit comprises a proximal conduit, a valve connecting conduit and a distal conduit. The present invention also discloses a preparation method thereof. The limiting expandable aortic valve conduit provided by the present invention is used for Bentall type surgery, and after the artificial biological valve dysfunctions, the limiting expandable aortic valve conduit provided by the present invention provides conditions for placing the artificial biological valve with the same size as the original valve in an interventional mode, namely, after the biological valve dysfunctions, the valve with the same size as the original valve can be placed in an interventional mode, and a patient's chest does not need to be opened to replace the biological valve with the same size.

## Description

### Technical Field

The present invention belongs to the technical field of biomedical materials, and particularly relates to a limiting expandable aortic valve conduit and preparation thereof.

### Background Art

The surgeries used to replace aortic artificial vessels and aortic valves include Bentall surgery, Mini-Bentall surgery, Cabrol surgery, Wheat's surgery, David surgery, Ross surgery and the like (hereinafter collectively referred to as "Bentall type surgery"), wherein the Bentall surgery was reported by a cardiac surgery expert named Bentall and DeBoNo in the earliest 1968, and thus it is called as "Bentall surgery". David surgery is generally referred to as Valve-Sparing Aortic Root Replacement (VSRR). Cabrol surgery is an improved surgery based on the Bentall surgery. Wheat's surgery is an aortic valve and ascending aortic replacement that retains the aortic sinus. This surgery was designed and completed by Wheat et al. in 1964.

For Bentall type surgery requiring valve replacement, heart valves used in aortic valve conduits are divided into mechanical valves and biological valves, and the mechanical valves are artificial valves made of non-metallic materials and metallic materials. The biological valve refers to an artificial heart valve manufactured by processing materials from the body of other animals (such as bovine pericardium and porcine pericardium). Mechanical valves have good durability, but patients require lifelong anticoagulant therapy. Biological valve decay (such as calcification) occurs after 10-15 years after biological valve replacement, the problem of aortic valve stenosis or aortic valve insufficiency occurs, and valve replacement is needed again.

The aortic valve conduit in the prior art has the following problems: (1) the suture edge of the artificial heart valve is connected with the left ventricular outflow channel, and after the biological valve dysfunctions, when the valve is replaced again, the chest needs to be opened for the second time to replace valve, or only the artificial heart valve with the size smaller than the previous size can be intervened in an intervention manner; (2) most of the conduit is made of polyester, the conduit is coated with a coating for preventing bleeding, preventing water permeability and increasing the endothelialization rate, and this coating cannot adapt to the high pulsation pressure of the aorta (especially the aortic root) and it is easy to fall off, and the bleeding and leakage prevention effects are required to be further improved; (3) poor biocompatibility, slow endothelialization rate, and inability to rapidly endothelialize can lead to hemolysis or thrombosis, and long-term local calcification; and (4) the thickness of the conduit is relatively large (> 0.5mm), resulting in insufficient surgical operability and compliance, and poor mechanical properties.

### Summary

The limiting expandable aortic valve conduit provided by the present invention is used for Bentall type surgery (which may include classical Bentall surgery, improved Bentall surgery, Mini-Bentall surgery, Cabrol surgery, Wheat's surgery, David surgery, Ross surgery, etc.), and after the artificial biological valve in the limiting expandable aortic valve conduit dysfunctions, the limiting expandable aortic valve conduit provided by the present invention provides conditions for placing the artificial biological valve with the same size as the original valve in an interventional manner, without the need to open the patient's chest to replace the artificial biological valve in a surgical manner, which greatly reduces the damage to the patient, and is particularly suitable for the patient without the condition of opening the chest to perform secondary valve replacement treatment.

The limiting expandable aortic valve conduit provided by the present invention is prepared from an aortic valve preliminary conduit attached with a collagen fiber coating. In the process of completing the present invention, inventor firstly optimizes the preparation method of the collagen fiber coating, then adopts the optimized preparation method to coat the aortic valve preliminary conduit to obtain the aortic valve preliminary conduit attached with the collagen fiber coating, and then prepares the aortic valve preliminary conduit which can bear high pulsation pressure, and the suture is not prone to falling off, so that the anti-bleeding effect is good, the endothelialization rate is high, the coating is stable, the degradation resistance is achieved, the biocompatibility is good, the mechanical property is excellent, and the limiting expandable aortic valve conduit is good in compliance.

The aortic valve preliminary conduit attached with the collagen fiber coating is obtained through two chemical crosslinking steps, including first chemical crosslinking and second chemical crosslinking, wherein the first chemical crosslinking is to crosslink collagen molecules or collagen fibers to obtain collagen fibers subjected to the first chemical crosslinking, and the second crosslinking is to attach the collagen fibers subjected to the first chemical crosslinking to internal gaps of the aortic valve preliminary conduit and internal and external surfaces of the aortic valve preliminary conduit to form the collagen fiber coating.

In the research process, the inventor unexpectedly find that the melting point of the collagen fiber coating and the melting point of the collagen fiber subjected to the first chemical crosslinking need to meet a certain difference relationship, and the aortic valve preliminary conduit with stable coating and good anti-bleeding effect can be obtained by optimizing and adjusting the difference value of the replace of the melting point of the collagen.

In addition, the inventor has unexpectedly found that by controlling the suture manner, the maximum circumferential tensile elongation, the elastic deformation rate, the maximum tensile force, the elastic deformation rate accounts for the value of the maximum circumferential tensile elongation, and the ratio of the corrugated width to the corrugated height, it is possible to effectively control the bleeding situation at the joint of the proximal conduit and the left ventricular outflow channel, the joint of the proximal conduit and the valve connecting conduit, and the joint of the valve connecting conduit and the distal conduit.

As an aspect of the present invention, it relates to a limiting expandable aortic valve conduit, comprising an aortic valve preliminary conduit attached with a collagen fiber coating, wherein the aortic valve preliminary conduit comprises a proximal conduit, a valve connecting conduit and a distal conduit;
by performing second chemical crosslinking on collagen fibers which have been subjected to first chemical crosslinking to have a melting point of 68.54-71.24°C; the collagen fiber coating is attached to internal gaps and surfaces of the aortic valve preliminary conduit; the melting point of the collagen fiber coating is 77.59-79.84°C, and the difference between the melting point of the collagen fiber coating and the melting point of the collagen fibers subjected to first chemical crosslinking ranges from 7.39-11.30°C.

Preferably, in a natural state of the aortic valve preliminary conduit, an axial length of the proximal conduit is not less than 5mm, and the proximal conduit comprises at least 2 corrugations in an axial direction; an axial length of the valve connecting conduit is 23-29mm, and the valve connecting conduit comprises 24-36 corrugations in a circumferential direction; an axial length of the distal conduit is not less than 70mm, and the distal conduit comprises at least 30 corrugations in an axial direction.

Preferably, the proximal conduit, the valve connecting conduit and the distal conduit are made of polyester corrugated conduits of a same specification, and a ratio of a corrugated width to a corrugated height of the polyester corrugated conduits is 1.24-1.36.

Preferably, a maximum circumferential tensile elongation range of the limiting expandable aortic valve conduit of the distal conduit is 2.2-4.8%, an elastic deformation rate range is 1.0-2.3%, the value of the elastic deformation rate in the maximum circumferential tensile elongation range is 38-68%, and a maximum tensile force range of the distal conduit is 13.5-55.9N.

Preferably, an outer diameter of a maximum bulging portion of the valve connecting conduit ranges from 28-38mm, and an outer diameter of a joint at two ends of the valve connecting conduit ranges from 24-33mm.

Preferably, further comprising a limiting expandable biological valve, wherein the limiting expandable biological valve is sewn on an inner surface of the valve connecting conduit, or the limiting expandable biological valve is sewn on an inner surface of a joint of the valve connecting conduit and the proximal conduit.

Preferably, further comprising a valve holder connected to the limiting expandable biological valve.

Preferably, two symmetrically arranged knotting protrusions are integrally connected to the rod body of the valve holder, and a suture cutting groove is formed between the two knotting protrusions.

Preferably, a joint of the proximal conduit/distal conduit and the valve connecting conduit is folded outward, an end portion of the valve connecting conduit is respectively sleeved on an outer side of the proximal conduit/distal conduit, and sutures pass back and forth through inner and outer side walls of the joint of the valve connecting conduit and the proximal conduit/distal conduit.

As another aspect of the present invention, it relates to a method for preparing a limiting expandable aortic valve conduit, comprising:
Step 1: cutting and connecting:
   selecting a polyester corrugated conduit with a corrugated width of 1.24-1.89mm, a corrugated height of 0.93-1.44mm, and a ratio of the corrugated width to the corrugated height of 1.24-1.36, cutting the polyester corrugated conduit in an axial direction of the polyester corrugated conduit, and cutting the polyester corrugated conduit to obtain a distal conduit, a valve intermediate conduit and a proximal conduit; and cutting the valve intermediate conduit along the axial direction of the valve intermediate conduit to obtain a rectangular corrugated conduit material, arranging the corrugations along the axial direction of the whole limiting expandable aortic valve conduit, and then sewing into a valve connecting conduit;
   obtaining an aortic valve preliminary conduit by sequentially connecting a proximal conduit, a valve connecting conduit and a distal conduit with sutures;
Step 2: attaching a collagen fiber coating on the internal gaps and the inner and outer surfaces of the aortic valve preliminary conduit:
   S1: preparing collagen fibers;
   S2: obtaining a collagen fiber having a melting point of 68.54-71.24°C through first chemical crosslinking by using biologically derived collagen as raw material and glutaraldehyde as crosslinking agent;
   S3: spraying the collagen fiber subjected to the first chemical crosslinking obtained in step S2 on the polyester corrugated conduit, and drying to obtain the polyester corrugated conduit adsorbed with the collagen fiber, wherein the spraying density of the collagen fiber subjected to the first chemical crosslinking on the surface of the polyester corrugated conduit is 1.5-6mg/cm²;
   S4: immersing the polyester corrugated conduit adsorbed with the collagen fiber in a crosslinking agent solution to obtain the polyester corrugated conduit immersed with the crosslinking agent adsorbed with the collagen fiber, wherein the crosslinking agent solution is a compound solution of N-hydroxysuccinimide and 1-(3-dimethylaminopropyl)-3-ethylcarbodiimide hydrochloride, the compound solution is compounded according to a mass ratio of 1:1, and the concentration is 27-40mg/mL;
   S5: immersing the polyester corrugated conduit obtained in step S4 into a collagen fiber solution of 3.5-5.5mg/mL of the collagen fiber subjected to the first chemical crosslinking so as to perform second chemical crosslinking, standing for 2 hours or more, and drying to obtain a polyester biological patch attached with a collagen fiber coating, wherein the melting point of the collagen fiber coating is 77.59-79.84°C;
Step 3: drying the limiting expandable biological valve to obtain a dried limiting expandable biological valve;
Step 4: assembling the valve holder, the dried limiting expandable biological valve and the aortic valve preliminary conduit to obtain a limiting expandable aortic valve conduit.

The collagen fiber coating of the limiting expandable aortic valve conduit provided by the present invention is stable, can bear the high pulsation pressure of the aorta (especially the aortic root), is not prone to falling off, is good in anti-bleeding effect, is not prone to degradation, is good in biocompatibility, is good in compliance and is excellent in mechanical property, and is used for replacing the aortic artificial blood vessel and the aortic valve in a Bentall type surgery.

According to the Bentall type surgery with limiting expandable aortic valve conduit provided by the present invention, after the artificial biological valve dysfunctions, the limiting expandable aortic valve conduit provided by the present invention provides conditions for placing the artificial biological valve with the same size as the original valve in an interventional mode, namely, after the biological valve dysfunctions, the valve with the same size as the original valve can be placed in an interventional mode, and the patient's chest does not need to be opened to replace the biological valve with the same size.

The inflow end of the proximal conduit of the limiting expandable aortic valve conduit is connected with the left ventricular outflow channel of a patient, and when the limiting expandable biological valve is expanded, many factors at the left ventricular outflow channel do not need to be considered. The valve seat of the limiting expandable biological valve is sewn in the limiting expandable aortic valve conduit, and conditions can be provided for expanding the limiting expandable biological valve by optimizing related parameters of the proximal conduit, the valve connecting conduit and the distal conduit. For a small aortic valve patient, in order to avoid the problem of prosthesispatient mismatch (PPM), the proximal conduit is directly cut open and the aortic root is enlarged.

### Brief Description of Drawings

FIG. 1 is a schematic diagram of the structure of a limiting expandable aortic valve conduit provided by examples 1-3.
FIG. 2 is a schematic diagram of the structure of a polyester corrugated conduit, wherein A is a schematic diagram of the structure of the polyester corrugated conduit; and B is a partial enlarged view of C in A.
FIG. 3 is a schematic diagram of the structure of a valve holder.
FIG. 4 is a schematic diagram of the structure of an aortic valve preliminary conduit in examples 1-3.
FIG. 5 is a sewing manner of a joint of a proximal conduit and a valve connecting conduit and a joint of the valve connecting conduit and a distal conduit in examples 1-3.
FIGS. 6A-6D are schematic diagrams of the structure of a limiting expandable biological valve.
FIG. 7 is a schematic diagram of the structure of a limiting expandable aortic valve conduit provided by examples 4-7.
FIG. 8 is a top view of a limiting expandable aortic valve conduit provided by examples 4-7.
FIG. 9 is a perspective view of a mold in examples 4-7.
FIG. 10 is a schematic diagram of the structure of an aortic valve preliminary conduit in examples 8-11.
FIG. 11 is a schematic diagram of the structure of an aortic valve preliminary conduit in example 12.

Reference numerals: aortic valve preliminary conduit 1, proximal conduit 2, valve connecting conduit 3, distal conduit 4, expandable sinus connecting conduit 5, wavy wiring 6, valve holder 7, suture cutting groove 8, threading hole 9, polyester corrugated conduit 10, identification line 12, mold 13, valve leaflet 14, valve frame 15, valve seat 16, polyester sleeve with suture edge 17, limiting expandable biological valve 18.

### Detailed Description

On the basis of the actual application scene requirements of the Bentall type surgery, aiming at the physiological environment of the aorta and the aortic root, the limiting expandable aortic valve conduit is provided, which can bear high pulsation pressure and the suture is not easy to fall off, the anti-bleeding effect is good, the endothelialization rate is high, the coating is stable, the degradation resistance is good, the biocompatibility is good, the mechanical property is excellent, and the compliance is good. After the artificial biological valve dysfunctions, the limiting expandable aortic valve conduit provided by the present invention provides conditions for placing the artificial biological valve with the same size as the original valve in an interventional mode, and the patient's chest does not need to be opened to replace the artificial biological valve in a surgical manner.

In the process of completing the present invention, the inventor firstly optimizes the preparation method of the collagen fiber coating, and in the optimization process, it is found that when the melting point of the collagen fiber subjected to first chemical crosslinking (denoted as "melting point 1") is 68.54-71.36°C, the melting point of the collagen fiber coating (denoted as "melting point 2") is 76.92-80.98°C, and the difference between the melting point 2 and the melting point 1 is within the range of 7.42-11.33°C, the collagen fiber coating is stable and the anti-bleeding effect is better.

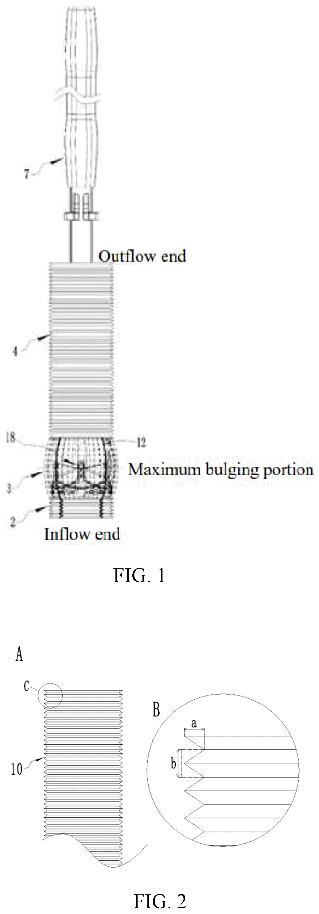

As shown in FIG. 1 to FIG. 11, the limiting expandable aortic valve conduit provided by the present invention comprises a proximal conduit 2, a valve connecting conduit 3 and a distal conduit 4, and the inventor firstly prepares a polyester corrugated conduit into the proximal conduit 2, the valve connecting conduit 3 and the distal conduit 4, and sews in sequence to obtain an aortic valve preliminary conduit 1, selects a preparation method with a stable collagen fiber coating and a better anti-bleeding effect to coat the aortic valve preliminary conduit 1, and then determines the melting point range of the collagen fiber coating as follows: the melting point 2 is 76.93-80.97°C, the melting point 1 is 68.54-71.36°C, the difference between the melting point 2 and the melting point 1 is 7.39-11.30°C, and finally connects the aortic valve preliminary conduit 1, the valve holder 7 and the dried limiting expandable biological valve 18 to obtain the limiting expandable aortic valve conduit.

An total water permeability experiment is performed on the limiting expandable aortic valve conduit, and it is found that the total water permeability at the joint of the proximal conduit 2 and the valve connecting conduit 3 and the joint of the valve connecting conduit 3 and the distal conduit 4 is relatively large, and by comparison, it can be found that the total water permeability of the limiting expandable aortic valve conduit with the melting point 2 of 77.59-79.84°C, the melting point 1 of 68.54-71.24°C, the difference between the melting point 2 and the melting point 1 of 7.39-11.30°C, the corrugated width (b in FIG. 2B) of 1.24-1.89mm, the corrugated height (a in FIG. 2B) of 0.93-1.44mm, and the ratio of b/a (the ratio of the corrugated width (b in FIG. 2B) to the corrugated height (a in FIG. 2B)) of 1.24-1.36 is relatively small, indicating good anti-bleeding effect.

Animal experiments are carried out on the limiting expandable aortic valve conduit, and it was found that the shear resistance of the limiting expandable aortic valve conduit' distal conduit with the maximum circumferential tensile elongation range of 2.2-4.8%, the elastic deformation rate range of 1.0-2.3%, the value of the elastic deformation rate in the maximum circumferential tensile elongation range of 38-68%, the maximum traction force range of 13.5-55.9N is high, no kinking phenomenon occurs, bleeding at the joint of the proximal conduit and the left ventricle outflow channel can be prevented, and the mechanical property is excellent. The finally obtained relevant parameters of the limiting expandable aortic valve conduit are as follows:
The melting point 2 is 77.59-79.84°C, the melting point 1 is 68.54-71.24°C, and the difference between the melting point 2 and the melting point 1 is 7.39-11.30°C; the maximum circumferential tensile elongation range of the distal conduit is 2.2-4.8%, the elastic deformation rate range of the distal conduit is 1.0-2.3%, the value of the elastic deformation rate in the maximum circumferential tensile elongation range is 38-68%, and the maximum tensile force range of the distal conduit is 13.5-55.9N; the corrugated width (b in FIG. 2B) is 1.24-1.89mm, and the corrugated height (a in FIG. 2B) is 0.93-1.44mm, and the ratio of b/a (the ratio of the corrugated width (b in FIG. 2B) to the corrugated height (a in FIG. 2B)) is 1.24-1.36.

### First: Experiments 1-60: Optimization of the preparation method of the conduit collagen fiber coating

### 1: Preparation method of the collagen fiber coating

### S1: Preparation of pig source type I collagen fibers

(1) Animal derived tissue pretreatment: after scraping off the surface fat of fresh pig skin tissue, soak it in acetone solution at room temperature for 16 hours, wash it 3-5 times with deionized water to remove excess acetone, then place the material in a 2.0M NaOH solution and stir at room temperature for 16 hours, soak it in deionized water after treatment, and clean the material until neutral. (2) Preparation of collagen molecule crude extract: the cleaned material is crushed in 0.5M acetic acid, homogenized, and the homogenized tissue is subjected to collagen molecule extraction at a ratio of 20:1 (tissue weight/pepsin weight), the temperature is controlled at 18°C and stirred for 72 hours. Centrifuge the extract to collect the supernatant, stir evenly, and then perform clarification filtration to control turbidity below 30, obtaining crude collagen molecule extract. (3) Purification: Use a tangential flow filtration system to ultrafiltration the crude extract of collagen molecules, concentrate it to 3mg/ml, and then dialyze it with 20mM acetic acid solution at a volume of 20 times to obtain collagen molecules. (4) Collagen molecular fibrosis: Take a certain volume of purified collagen molecules and add 1/10 volume of phosphate buffer solution (0.2M Na₂HPO₄: 0.2M NaH₂PO₄=7:3, v: v). The pH was adjusted to 7.0, and placed at 30°C for 6 hours to obtain collagen fibers. In the Steps (1)-(4), collagen fibers obtained by using biogenic collagen as a raw material, and any means disclosed in the prior art may be used in steps (1)-(4), as long as the corresponding technical purpose can be achieved.

### S2: First chemical crosslinking:

The collagen fibers obtained in steps (1)-(4) were added to glutaraldehyde aqueous solutions with different concentrations, mixed uniformly, stirred overnight at 30°C, centrifuged to collect precipitates, washed with pH7.0 phosphate buffer solution for 2-3 times to obtain collagen fibers subjected to first chemical crosslinking. The concentration range of glutaraldehyde aqueous solution is 0.004%-0.035%.

The concentration of glutaraldehyde aqueous solution used in experiments 1-10 is shown in Table 1 below. The difference between the experiments 1-10 is that the concentrations of glutaraldehyde aqueous solutions are different. The melting point of the collagen fiber subjected to the first chemical crosslinking (denoted as "melting point 1") obtained in experiments 1-10 was measured by differential scanning calorimetry (DSC), specifically, the collagen fiber subjected to the first chemical crosslinking obtained in experiments 1-10 was transferred into a test aluminum crucible of a differential scanning calorimeter, sealed in the differential scanning calorimeter, heated at a temperature of 10K/min under a nitrogen atmosphere, and subjected to scanning measurement at a temperature rise range of 25-100°C (the temperature range covers the melting point range of the substance). It can be seen from Table 1 that the melting point 1 of the collagen fiber subjected to the first chemical crosslinking obtained in experiments 1-10 is 68.54-71.36°C. The main differences and measured melting points 1 for experiments 1-10 are shown in Table 1:

**Table 1: Main differences and measured melting points 1 for experiments 1-10**

| Groups | Concentrations of glutaraldehyde aqueous solution | Melting points 1 (°C) | Groups | Concentrations of glutaraldehyde aqueous solution | Melting points 1 (°C) |
|---|---|---|---|---|---|
| Experiment 1 | 0.004% | 68.54±0.13 | Experiment 6 | 0.016% | 70.77±0.02 |
| Experiment 2 | 0.009% | 69.82±0.10 | Experiment 7 | 0.018% | 70.83±0.12 |
| Experiment 3 | 0.011% | 70.17±0.05 | Experiment 8 | 0.025% | 71.08±0.01 |
| Experiment 4 | 0.012% | 70.36±0.09 | Experiment 9 | 0.03% | 71.24±0.03 |
| Experiment 5 | 0.014% | 70.61±0.02 | Experiment 10 | 0.035% | 71.36±0.02 |

### S3: Preparation of coating

Select the following parameters for the polyester corrugated conduit: the inner diameter is 20-22mm, the outer diameter is 23-25mm, and the thickness is 0.15-0.45 mm, the corrugated height (a in FIG. 2B) is 0.5-2.0mm, and the corrugated width (b in FIG. 2B) is 0.5-3.0mm. The collagen fibers subjected to the first chemical crosslinking obtained in step S2 are uniformly sprayed on the inner surface and the outer surface of the polyester corrugated conduit at a spraying density of 1-6mg/cm² (that is, 1-6mg is uniformly sprayed on the inner surface and the outer surface of the conduit per square centimeter), and after spraying, the collagen fibers are dried in an oven at 30-35°C to obtain the conduit adsorbed with the collagen fibers.

**Table 2: The main differences in S3 for experiments 11-60**

| Groups | Collagen fiber sources subjected to the first chemical crosslinking in S3 | Spraying density in S3 (mg/cm²) | Drying temperature in S3 | Corrugated width (b in Figure 2B) | Corrugated height (a in Figure 2B) | b/a |
|---|---|---|---|---|---|---|
| Experiment 11 | Prepared in Experiment 1 | 1 | 30 | 1.07±0.01 | 0.81±0.05 | 1.32 |
| Experiment 12 | Prepared in Experiment 1 | 1.5 | 31 | 1.48±0.02 | 1.13±0.02 | 1.31 |
| Experiment 13 | Prepared in Experiment 1 | 2.5 | 32 | 1.56±0.01 | 1.26±0.04 | 1.24 |
| Experiment 14 | Prepared in Experiment 1 | 5 | 33 | 1.92±0.11 | 1.35±0.01 | 1.42 |
| Experiment 15 | Prepared in Experiment 1 | 5.5 | 32 | 1.89±0.16 | 1.44±0.04 | 1.31 |
| Experiment 16 | Prepared in Experiment 2 | 1 | 30 | 0.51±0.01 | 0.84±0.04 | 0.61 |
| Experiment 17 | Prepared in Experiment 2 | 2 | 31 | 1.11±0.03 | 1.52±0.01 | 0.73 |
| Experiment 18 | Prepared in Experiment 2 | 2.5 | 32 | 1.65±0.03 | 1.25±0.01 | 1.32 |
| Experiment 19 | Prepared in Experiment 2 | 4.5 | 33 | 1.24±0.02 | 0.93±0.04 | 1.33 |
| Experiment 20 | Prepared in Experiment 2 | 6 | 35 | 1.33±0.01 | 1.96±0.04 | 0.68 |
| Experiment 21 | Prepared in Experiment 3 | 3 | 30 | 1.29±0.02 | 1.99±0.01 | 0.65 |
| Experiment 22 | Prepared in Experiment 3 | 4 | 31 | 1.08±0.11 | 1.64±0.02 | 0.66 |
| Experiment 23 | Prepared in Experiment 3 | 3.5 | 32 | 1.37±0.04 | 1.98±0.04 | 0.69 |
| Experiment 24 | Prepared in Experiment 3 | 6 | 33 | 0.91±0.04 | 1.11±0.04 | 0.82 |
| Experiment 25 | Prepared in Experiment 3 | 6 | 35 | 0.69±0.12 | 0.74±0.02 | 0.93 |
| Experiment 26 | Prepared in Experiment 4 | 1.5 | 30 | 0.68±0.15 | 0.65±0.02 | 1.05 |
| Experiment 27 | Prepared in Experiment 4 | 3 | 31 | 1.38±0.02 | 1.03±0.01 | 1.34 |
| Experiment 28 | Prepared in Experiment 4 | 3 | 32 | 1.27±0.02 | 0.94±0.01 | 1.35 |
| Experiment 29 | Prepared in Experiment 4 | 6 | 33 | 1.33±0.02 | 1.22±0.02 | 1.09 |
| Experiment 30 | Prepared in Experiment 4 | 6 | 35 | 1.69±0.05 | 1.48±0.03 | 1.14 |
| Experiment 31 | Prepared in Experiment 5 | 1 | 30 | 1.29±0.04 | 1.08±0.01 | 1.19 |
| Experiment 32 | Prepared in Experiment 5 | 2 | 31 | 1.43±0.02 | 1.16±0.01 | 1.23 |
| Experiment 33 | Prepared in Experiment 5 | 3.5 | 32 | 1.54±0.04 | 1.13±0.03 | 1.36 |
| Experiment 34 | Prepared in Experiment 5 | 4.5 | 33 | 1.57±0.01 | 1.29±0.03 | 1.22 |
| Experiment 35 | Prepared in Experiment 5 | 5.5 | 33 | 1.7±0.03 | 1.37±0.02 | 1.24 |
| Experiment 36 | Prepared in Experiment 6 | 1 | 30 | 2.25±0.02 | 1.57±0.01 | 1.43 |
| Experiment 37 | Prepared in Experiment 6 | 2.5 | 31 | 2.48±0.05 | 1.71±0.04 | 1.45 |
| Experiment 38 | Prepared in Experiment 6 | 3.5 | 32 | 2.41±0.13 | 1.66±0.05 | 1.45 |
| Experiment 39 | Prepared in Experiment 6 | 4.5 | 33 | 2.47±0.04 | 1.69±0.02 | 1.46 |
| Experiment 40 | Prepared in Experiment 6 | 5.5 | 31 | 2.53±0.03 | 1.72±0.03 | 1.47 |
| Experiment 41 | Prepared in Experiment 7 | 1.5 | 30 | 2.14±0.01 | 1.42±0.03 | 1.51 |
| Experiment 42 | Prepared in Experiment 7 | 3 | 31 | 1.85±0.01 | 1.22±0.01 | 1.52 |
| Experiment 43 | Prepared in Experiment 7 | 3.5 | 32 | 2.51±0.12 | 1.57±0.02 | 1.60 |
| Experiment 44 | Prepared in Experiment 7 | 5.5 | 33 | 2.58±0.01 | 1.52±0.06 | 1.70 |
| Experiment 45 | Prepared in Experiment 7 | 6 | 35 | 2.41±0.03 | 1.37±0.02 | 1.76 |
| Experiment 46 | Prepared in Experiment 8 | 1.5 | 30 | 2.04±0.04 | 1.15±0.01 | 1.77 |
| Experiment 47 | Prepared in Experiment 8 | 2.5 | 31 | 2.14±0.04 | 1.23±0.02 | 1.74 |
| Experiment 48 | Prepared in Experiment 8 | 3.5 | 32 | 2.63±0.12 | 1.48±0.01 | 1.78 |
| Experiment 49 | Prepared in Experiment 8 | 6 | 33 | 2.55±0.04 | 1.33±0.03 | 1.92 |
| Experiment 50 | Prepared in Experiment 8 | 5.5 | 35 | 2.57±0.01 | 1.32±0.04 | 1.95 |
| Experiment 51 | Prepared in Experiment 9 | 1.5 | 30 | 2.57±0.14 | 1.27±0.02 | 2.02 |
| Experiment 52 | Prepared in Experiment 9 | 2 | 31 | 2.18±0.03 | 1.05±0.01 | 2.08 |
| Experiment 53 | Prepared in Experiment 9 | 3.5 | 32 | 2.26±0.14 | 1.08±0.05 | 2.09 |
| Experiment 54 | Prepared in Experiment 9 | 4 | 33 | 1.58±0.01 | 1.16±0.03 | 1.36 |
| Experiment 55 | Prepared in Experiment 9 | 5.5 | 34 | 2.52±0.11 | 1.12±0.02 | 2.25 |
| Experiment 56 | Prepared in Experiment 10 | 1.5 | 30 | 2.73±0.03 | 1.21±0.03 | 2.26 |
| Experiment 57 | Prepared in Experiment 10 | 3 | 31 | 2.55±0.01 | 1.11±0.01 | 2.30 |
| Experiment 58 | Prepared in Experiment 10 | 3.5 | 32 | 1.62±0.01 | 1.15±0.06 | 1.41 |
| Experiment 59 | Prepared in Experiment 10 | 4.5 | 33 | 1.11±0.01 | 0.84±0.05 | 1.32 |
| Experiment 60 | Prepared in Experiment 10 | 5.5 | 30 | 2.62±0.06 | 1.02±0.01 | 2.57 |

It can be seen from the above Table that the corrugated height (a in FIG. 2B) of the conduit obtained in experiments 11-60 is 0.65-1.99mm, the corrugated width (b in FIG. 2B) is 0.51-2.73mm, and the b/a range is 0.61-2.57.

S4: Fully immersing the conduit adsorbed with the collagen fiber in a second crosslinking agent solution, and standing for 20 minutes at room temperature to obtain the conduit immersed with the crosslinking agent and adsorbed with the collagen fiber. The preparation method of the second crosslinking agent solution comprises the following steps: weighing N-hydroxysuccinimide (NHS) and 1-(3-dimethylaminopropyl)-3-ethylcarbodiimide hydrochloride (EDC) according to a mass ratio of 1:1 in a centrifuge conduit, and adding a pH7.2 phosphate (PBS) solution to obtain the crosslinking agent solution, wherein the concentrations of NHS and EDC are 10-60mg/mL.

**Table 3: Concentrations in S4 for experiments 11-60**

| Groups | Concentrations of crosslinking agent in S4 (mg/mL) | Groups | Concentrations of crosslinking agent in S4 (mg/mL) | Groups | Concentrations of crosslinking agent in S4 (mg/mL) |
|---|---|---|---|---|---|
| Experiment 11 | 10 | Experiment 28 | 35 | Experiment 45 | 47 |
| Experiment 12 | 27 | Experiment 29 | 46 | Experiment 46 | 15 |
| Experiment 13 | 38 | Experiment 30 | 54 | Experiment 47 | 23 |
| Experiment 14 | 48 | Experiment 31 | 12 | Experiment 48 | 44 |
| Experiment 15 | 31 | Experiment 32 | 15 | Experiment 49 | 41 |
| Experiment 16 | 17 | Experiment 33 | 33 | Experiment 50 | 59 |
| Experiment 17 | 12 | Experiment 34 | 45 | Experiment 51 | 14 |
| Experiment 18 | 29 | Experiment 35 | 52 | Experiment 52 | 15 |
| Experiment 19 | 37 | Experiment 36 | 11 | Experiment 53 | 28 |
| Experiment 20 | 60 | Experiment 37 | 15 | Experiment 54 | 36 |
| Experiment 21 | 18 | Experiment 38 | 32 | Experiment 55 | 53 |
| Experiment 22 | 57 | Experiment 39 | 42 | Experiment 56 | 13 |
| Experiment 23 | 58 | Experiment 40 | 25 | Experiment 57 | 34 |
| Experiment 24 | 55 | Experiment 41 | 49 | Experiment 58 | 39 |
| Experiment 25 | 56 | Experiment 42 | 43 | Experiment 59 | 40 |
| Experiment 26 | 16 | Experiment 43 | 50 | Experiment 60 | 20 |
| Experiment 27 | 30 | Experiment 44 | 51 | | |

### S5: Second chemical crosslinking

The first chemically crosslinked collagen fiber obtained from S2 was formulated into a collagen fiber solution with a concentration of 1-8.5mg/mL using pH7.2 phosphate (PBS) solution. The conduit with collagen fibers adsorbed with the crosslinking agent is immersed in a collagen fiber solution, and is allowed to stand at room temperature for 2 hours or more (preferably, stand overnight), and is fully dried at 30-35°C, at this time, the collagen fibers subjected to two times of chemical crosslinking form a collagen fiber coating, which exists in the internal gaps of the conduit and the internal and external surfaces of the conduit to obtain the conduit with the collagen fiber coating attached thereto.

The melting point of the collagen fiber coating (denoted as "melting point 2") was determined by differential scanning calorimetry DSC. Specifically, the conduit attached with the collagen fiber coating and the conduit raw material (or referred to as a polyester corrugated conduit) are transferred into a test aluminum crucible of a differential scanning calorimeter, sealed in the differential scanning calorimeter, heated at a temperature of 10K/min under a nitrogen atmosphere, and scanned and determined under the condition that the heating range is 25-100°C (the temperature range covers the melting point range of the substance). The melting point of the collagen fiber coating is the melting point of the collagen fiber coating with the background peak of the conduit raw material removed. It can be seen from Table 4 that when the melting point 1 is 68.54-71.36°C, the melting point 2 of the conduit collagen fiber coating obtained in experiments 11-60 is 71.36-85.53°C, and the difference between the melting point 2 and the melting point 1 is 0.65-15.71°C.

**Table 4: Differences and related melting points in S5 for experiments 11-60**

| Groups | Concentration of collagen fiber solution in s5 (mg/ml) | Drying temperature in S5 (°C) | Melting point 2 (°C) | Melting point 1 (°C) | Difference between melting point 2 and melting point 1 (°C) |
|---|---|---|---|---|---|
| Experiment 11 | 1 | 30 | 71.36±0.05 | 68.54±0.13 | 2.82 |
| Experiment 12 | 3.5 | 31 | 76.92±0.03 | 68.54±0.13 | 8.38 |
| Experiment 13 | 5.5 | 32 | 79.87±0.21 | 68.54±0.13 | 11.33 |
| Experiment 14 | 7 | 33 | 82.93±0.03 | 68.54±0.13 | 14.39 |
| Experiment 15 | 4 | 35 | 78.04±0.08 | 68.54±0.13 | 9.50 |
| Experiment 16 | 3 | 30 | 74.54±0.02 | 69.82±0.10 | 4.72 |
| Experiment 17 | 1.5 | 31 | 71.66±0.03 | 69.82±0.10 | 1.84 |
| Experiment 18 | 3.5 | 32 | 77.51±0.07 | 69.82±0.10 | 7.69 |
| Experiment 19 | 5 | 33 | 79.25±0.02 | 69.82±0.10 | 9.43 |
| Experiment 20 | 8.5 | 35 | 85.53±0.06 | 69.82±0.10 | 15.71 |
| Experiment 21 | 3 | 30 | 74.62±0.03 | 70.17±0.05 | 4.45 |
| Experiment 22 | 8.5 | 31 | 85.28±0.02 | 70.17±0.05 | 15.11 |
| Experiment 23 | 8.5 | 32 | 85.31±0.12 | 70.17±0.05 | 15.14 |
| Experiment 24 | 8 | 33 | 84.52±0.04 | 70.17±0.05 | 14.35 |
| Experiment 25 | 8 | 35 | 84.73±0.02 | 70.17±0.05 | 14.56 |
| Experiment 26 | 3 | 30 | 73.88±0.01 | 70.36±0.09 | 3.52 |
| Experiment 27 | 4 | 31 | 77.78±0.11 | 70.36±0.09 | 7.42 |
| Experiment 28 | 4.5 | 32 | 78.82±0.08 | 70.36±0.09 | 8.46 |
| Experiment 29 | 6.5 | 33 | 82.25±0.02 | 70.36±0.09 | 11.89 |
| Experiment 30 | 7.5 | 35 | 84.07±0.04 | 70.36±0.09 | 13.71 |
| Experiment 31 | 1 | 30 | 71.57±0.25 | 70.61±0.02 | 0.96 |
| Experiment 32 | 2.5 | 31 | 73.54±0.01 | 70.61±0.02 | 2.93 |
| Experiment 33 | 4.5 | 32 | 78.23±0.02 | 70.61±0.02 | 7.62 |
| Experiment 34 | 6.5 | 33 | 81.93±0.04 | 70.61±0.02 | 11.32 |
| Experiment 35 | 7 | 35 | 83.86±0.05 | 70.61±0.02 | 13.25 |
| Experiment 36 | 1 | 30 | 71.42±0.08 | 70.77±0.02 | 0.65 |
| Experiment 37 | 2.5 | 31 | 73.62±0.02 | 70.77±0.02 | 2.85 |
| Experiment 38 | 4 | 32 | 78.16±0.04 | 70.77±0.02 | 7.39 |
| Experiment 39 | 6 | 33 | 81.09±0.08 | 70.77±0.02 | 10.32 |
| Experiment 40 | 3.5 | 35 | 75.83±0.02 | 70.77±0.02 | 5.06 |
| Experiment 41 | 7 | 30 | 83.17±0.07 | 70.83±0.12 | 12.34 |
| Experiment 42 | 6 | 31 | 81.22±0.05 | 70.83±0.12 | 10.39 |
| Experiment 43 | 7 | 32 | 83.26±0.02 | 70.83±0.12 | 12.43 |
| Experiment 44 | 7 | 33 | 83.44±0.04 | 70.83±0.12 | 12.61 |
| Experiment 45 | 6.5 | 35 | 82.68±0.06 | 70.83±0.12 | 11.85 |
| Experiment 46 | 2 | 30 | 73.41±0.01 | 71.08±0.01 | 2.33 |
| Experiment 47 | 3.5 | 31 | 75.66±0.01 | 71.08±0.01 | 4.58 |
| Experiment 48 | 6 | 32 | 81.59±0.05 | 71.08±0.01 | 10.51 |
| Experiment 49 | 5.5 | 33 | 81.07±0.11 | 71.08±0.01 | 9.99 |
| Experiment 50 | 8.5 | 35 | 85.44±0.12 | 71.08±0.01 | 14.36 |
| Experiment 51 | 2 | 30 | 73.19±0.04 | 71.24±0.03 | 1.95 |
| Experiment 52 | 2.5 | 31 | 73.48±0.11 | 71.24±0.03 | 2.24 |
| Experiment 53 | 3.5 | 32 | 76.98±0.02 | 71.24±0.03 | 5.74 |
| Experiment 54 | 5 | 33 | 78.93±0.03 | 71.24±0.03 | 7.69 |
| Experiment 55 | 7.5 | 35 | 84.04±0.05 | 71.24±0.03 | 12.8 |
| Experiment 56 | 2 | 30 | 72.22±0.02 | 71.36±0.02 | 0.86 |
| Experiment 57 | 4.5 | 31 | 78.55±0.14 | 71.36±0.02 | 7.19 |
| Experiment 58 | 5.5 | 32 | 80.91±0.01 | 71.36±0.02 | 9.55 |
| Experiment 59 | 5.5 | 33 | 80.98±0.13 | 71.36±0.02 | 9.62 |
| Experiment 60 | 3.5 | 35 | 75.73±0.10 | 71.36±0.02 | 4.37 |

### S6: Drying

The method comprises the following steps: drying a conduit attached with a collagen fiber coating, specifically immersing the conduit attached with the collagen fiber coating in 100% glycerol, and fully replacing water molecules in the conduit attached with the collagen fiber coating with glycerol to obtain a dried conduit.

### 2: Falling off of conduit coating obtained in experiments 11-60

The stability of the coating is reflected by the collagen content and collagen infrared transmittance before and after the pulsating flow experiment. Specifically, the conduits prepared in the above experiments 11-60 were respectively subjected to a pulsating flow experiment, specifically, according to the ISO 5840 standard, the conduits were cut into sheet-shaped samples with a length of 4cm and a width of 1cm, and sewn on a pulsating flow platform; under standard physiological conditions, after pulsing for 90 days, the content of collagen and the infrared transmittance of collagen in the sheet-shaped samples before and after the pulsating flow test were compared, and the stability of the collagen fiber coating under pulsating flow was evaluated. The test results are shown in Table 5, and it can be seen from Table 5 that the collagen fiber coatings of the conduits obtained in experiments 12-13, 15, 18-19, 27-28, 33, 54, 58-59 are relatively stable.

**Table 5: Changes in the content of collagen coating before and after conduit pulsation obtained in experiments 11-60**

| Groups | Collagen content (mg/cm²) | | | Collagen infrared transmittance (%) | | |
|---|---|---|---|---|---|---|
| | before pulsation | after pulsation | Difference | before pulsation | after pulsation | Difference |
| Experiment 11 | 0.121±0.005 | 0.017±0.002 | 0.104 | 78.02±0.14 | 79.19±0.01 | 1.17 |
| Experiment 12 | 0.808±0.001 | 0.807±0.011 | 0.001 | 72.81±0.03 | 72.89±0.01 | 0.08 |
| Experiment 13 | 0.919±0.009 | 0.892±0.023 | 0.027 | 68.24±0.06 | 68.78±0.06 | 0.54 |
| Experiment 14 | 1.193±0.002 | 0.879±0.002 | 0.314 | 62.91±0.04 | 64.06±0.05 | 1.15 |
| Experiment 15 | 0.863±0.006 | 0.819±0.004 | 0.044 | 70.91±0.02 | 70.96±0.13 | 0.05 |
| Experiment 16 | 0.694±0.001 | 0.493±0.001 | 0.201 | 75.18±0.02 | 76.22±0.04 | 1.04 |
| Experiment 17 | 0.234±0.001 | 0.112±0.001 | 0.122 | 76.55±0.04 | 77.79±0.08 | 1.24 |
| Experiment 18 | 0.849±0.01 | 0.816±0.012 | 0.033 | 71.72±0.13 | 72.48±0.08 | 0.76 |
| Experiment 19 | 0.913±0.004 | 0.888±0.003 | 0.025 | 69.72±0.22 | 69.96±0.14 | 0.24 |
| Experiment 20 | 1.495±0.002 | 0.993±0.011 | 0.502 | 56.84±0.13 | 57.72±0.05 | 0.88 |
| Experiment 21 | 0.697±0.005 | 0.204±0.013 | 0.493 | 74.57±0.06 | 75.86±0.04 | 1.29 |
| Experiment 22 | 1.356±0.006 | 1.161±0.009 | 0.195 | 58.51±0.06 | 59.54±0.06 | 1.03 |
| Experiment 23 | 1.464±0.003 | 1.042±0.003 | 0.422 | 57.42±0.07 | 58.66±0.08 | 1.24 |
| Experiment 24 | 1.249±0.01 | 0.436±0.004 | 0.813 | 59.63±0.18 | 61.97±0.04 | 2.34 |
| Experiment 25 | 1.353±0.003 | 1.139±0.011 | 0.214 | 58.48±0.06 | 60.31±0.06 | 1.83 |
| Experiment 26 | 0.694±0.001 | 0.476±0.001 | 0.218 | 75.15±0.08 | 76.19±0.07 | 1.04 |
| Experiment 27 | 0.855±0.003 | 0.838±0.013 | 0.017 | 70.96±0.18 | 71.1±0.06 | 0.14 |
| Experiment 28 | 0.903±0.017 | 0.887±0.002 | 0.016 | 70.01±0.03 | 70.14±0.02 | 0.13 |
| Experiment 29 | 1.147±0.018 | 0.611±0.005 | 0.536 | 63.96±0.05 | 65.33±0.04 | 1.37 |
| Experiment 30 | 1.225±0.003 | 1.051±0.009 | 0.174 | 58.92±0.18 | 60.69±0.04 | 1.77 |
| Experiment 31 | 0.206±0.006 | 0.077±0.003 | 0.129 | 76.64±0.12 | 77.79±0.03 | 1.15 |
| Experiment 32 | 0.623±0.007 | 0.434±0.021 | 0.189 | 75.55±0.07 | 76.74±0.03 | 1.19 |
| Experiment 33 | 0.875±0.002 | 0.799±0.009 | 0.076 | 70.17±0.01 | 70.94±0.05 | 0.77 |
| Experiment 34 | 1.123±0.002 | 0.546±0.005 | 0.577 | 64.73±0.33 | 66.1±0.05 | 1.37 |
| Experiment 35 | 1.239±0.001 | 0.532±0.007 | 0.707 | 60.71±0.04 | 62.82±0.07 | 2.11 |
| Experiment 36 | 0.188±0.004 | 0.074±0.002 | 0.114 | 77.28±0.01 | 79.29±0.05 | 2.01 |
| Experiment 37 | 0.623±0.007 | 0.024±0.021 | 0.599 | 75.69±0.05 | 76.64±0.03 | 0.95 |
| Experiment 38 | 0.868±0.009 | 0.552±0.011 | 0.316 | 70.25±0.06 | 71.4±0.03 | 1.15 |
| Experiment 39 | 0.989±0.001 | 0.371±0.008 | 0.618 | 67.01±0.01 | 69.06±0.02 | 2.05 |
| Experiment 40 | 0.784±0.002 | 0.467±0.031 | 0.317 | 72.98±0.03 | 74.14±0.02 | 1.16 |
| Experiment 41 | 1.225±0.002 | 0.607±0.003 | 0.618 | 62.24±0.02 | 64.25±0.07 | 2.01 |
| Experiment 42 | 1.003±0.002 | 0.47±0.014 | 0.533 | 66.95±0.06 | 68.31±0.09 | 1.36 |
| Experiment 43 | 1.235±0.018 | 0.474±0.004 | 0.761 | 61.34±0.01 | 63.49±0.05 | 2.15 |
| Experiment 44 | 1.238±0.010 | 1.008±0.005 | 0.230 | 61.34±0.01 | 62.3±0.05 | 0.96 |
| Experiment 45 | 1.151±0.006 | 0.563±0.019 | 0.588 | 63.24±0.06 | 64.42±0.02 | 1.18 |
| Experiment 46 | 0.442±0.006 | 0.128±0.015 | 0.314 | 76.33±0.04 | 78.26±0.08 | 1.93 |
| Experiment 47 | 0.711±0.001 | 0.498±0.001 | 0.213 | 74.52±0.01 | 75.59±0.47 | 1.07 |
| Experiment 48 | 1.086±0.012 | 0.604±0.001 | 0.482 | 65.28±0.03 | 66.34±0.16 | 1.06 |
| Experiment 49 | 0.965±0.05 | 0.361±0.005 | 0.604 | 67.11±0.02 | 68.86±0.01 | 1.75 |
| Experiment 50 | 1.475±0.002 | 0.654±0.011 | 0.821 | 56.39±0.13 | 57.43±0.02 | 1.04 |
| Experiment 51 | 0.326±0.011 | 0.109±0.006 | 0.217 | 76.38±0.02 | 77.55±0.01 | 1.17 |
| Experiment 52 | 0.565±0.014 | 0.161±0.003 | 0.404 | 76.22±0.04 | 77.96±0.02 | 1.74 |
| Experiment 53 | 0.842±0.011 | 0.24±0.025 | 0.602 | 72.16±0.06 | 73.9±0.06 | 1.74 |
| Experiment 54 | 0.905±0.009 | 0.892±0.003 | 0.013 | 69.98±0.04 | 70.01±0.12 | 0.03 |
| Experiment 55 | 1.246±0.004 | 0.64±0.002 | 0.606 | 60.15±0.02 | 61.74±0.04 | 1.59 |
| Experiment 56 | 0.242±0.012 | 0.128±0.005 | 0.114 | 76.44±0.06 | 77.78±0.05 | 1.34 |
| Experiment 57 | 0.875±0.002 | 0.671±0.009 | 0.204 | 70.11±0.04 | 71.18±0.06 | 1.07 |
| Experiment 58 | 0.942±0.009 | 0.917±0.012 | 0.025 | 67.42±0.03 | 67.85±0.04 | 0.43 |
| Experiment 59 | 0.958±0.021 | 0.94±0.019 | 0.018 | 67.37±0.01 | 67.66±0.04 | 0.29 |
| Experiment 60 | 0.732±0.004 | 0.144±0.008 | 0.588 | 74.43±0.04 | 75.86±0.04 | 1.43 |

### 3: Total Water Permeability Experiment

The flow rate of water leakage through the conduit wall at 16kPa (120mmHg) pressure was measured as performed at YYT0500-2021. The specific test steps are:
Prior to testing, the conduit was wetted with clean filtered water at room temperature (or at a specified temperature). The conduit was tested in an implantable state. The distal end of the sample is sealed using a plug or adapter suitable for the inner diameter of the conduit. The proximal end of the conduit is connected to an adapter with a specific inner diameter to ensure sealing. The adapter and conduit are connected to a fixture for pressure generation and measurement. The intraluminal pressure of the sample is gradually increased, and the trapped air is discharged. The pressure was brought to 16.0kPa±0.3kPa (120mmHg±2mmHg), the pressure or flow was maintained stable, and the amount of water seeping through the walls within 60s was measured and the total water permeability was calculated. Total water permeability is expressed as ml/(cm²·min).

**Table 6: Total water permeability of conduit obtained in experiments 11-60**

| Groups | Total Water Permeability (ml/(cm²·min)) | Groups | Total Water Permeability (ml/(cm²·min)) | Groups | Total Water Permeability (ml/(cm²·min)) |
|---|---|---|---|---|---|
| Experiment 11 | 0.98±0.38 | Experiment 28 | 0.28±0.02 | Experiment 45 | 1.04±0.02 |
| Experiment 12 | 0.32±0.01 | Experiment 29 | 1.57±0.03 | Experiment 46 | 0.97±0.01 |
| Experiment 13 | 0.47±0.02 | Experiment 30 | 1.05±0.02 | Experiment 47 | 2.23±0.07 |
| Experiment 14 | 1.40±0.04 | Experiment 31 | 1.44±0.01 | Experiment 48 | 2.89±0.03 |
| Experiment 15 | 0.13±0.01 | Experiment 32 | 2.86±0.06 | Experiment 49 | 3.14±0.04 |
| Experiment 16 | 2.10±0.01 | Experiment 33 | 0.17±0.01 | Experiment 50 | 2.03±0.05 |
| Experiment 17 | 2.23±0.02 | Experiment 34 | 1.45±0.08 | Experiment 51 | 1.32±0.06 |
| Experiment 18 | 0.24±0.05 | Experiment 35 | 1.49±0.04 | Experiment 52 | 1.07±0.02 |
| Experiment 19 | 0.11±0.03 | Experiment 36 | 3.42±0.02 | Experiment 53 | 2.79±0.01 |
| Experiment 20 | 1.56±0.30 | Experiment 37 | 2.32±0.08 | Experiment 54 | 0.08±0.02 |
| Experiment 21 | 1.94±0.01 | Experiment 38 | 1.53±0.09 | Experiment 55 | 1.74±0.07 |
| Experiment 22 | 0.82±0.06 | Experiment 39 | 1.69±0.02 | Experiment 56 | 2.72±0.01 |
| Experiment 23 | 0.59±0.01 | Experiment 40 | 1.93±0.03 | Experiment 57 | 1.34±0.05 |
| Experiment 24 | 1.34±0.02 | Experiment 41 | 2.01±0.05 | Experiment 58 | 0.13±0.01 |
| Experiment 25 | 1.30±0.03 | Experiment 42 | 1.30±0.06 | Experiment 59 | 0.26±0.01 |
| Experiment 26 | 1.42±0.06 | Experiment 43 | 0.93±0.04 | Experiment 60 | 1.55±0.03 |
| Experiment 27 | 0.16±0.01 | Experiment 44 | 1.38±0.02 | | |

In summary, it can be seen that the total water permeability of the coatings of the conduits obtained in experiments 12-13, 15, 18-19, 27-28, 33, 54, 58-59 is low, and the total water permeability range is 0.08-0.47ml/(cm²·min).

By combining the coating falling off condition and the total water permeability condition, the inventor surprisingly finds that when the melting point 1 is 68.54-71.36°C, the melting point 2 is 76.92-80.98°C, the coating with the difference between the melting point 2 and the melting point 1 in the range of 7.42-11.33°C is more stable, the total water permeability is low, and the anti-bleeding effect is good.

Experiments 12-13, 15, 18-19, 27-28, 33, 54, 58-59, hereinafter referred to as 11 optimization experiments.

### Second: Examples 1-12: Limiting expandable aortic valve conduit

### 1: Structure of limiting expandable aortic valve conduit of examples 1-12

As shown in FIGS. 1-11, examples 1-12 provide a limiting expandable aortic valve conduit, which includes a proximal conduit 2, a valve connecting conduit 3, a distal conduit 4 and a limiting expandable biological valve 18.

The materials of the proximal conduit 2, the valve connecting conduit 3 and the distal conduit 4 are polyester corrugated conduits 10 of the same or different specifications, as shown in FIG. 2, relevant parameters of the polyester corrugated conduits 10 are as follows: inner diameter of 20-22mm, outer diameter of 23-25mm, thickness of 0.15-0.45mm, corrugated height (a in FIG. 2B) of 0.5-2.0mm, corrugated width (b in FIG. 2B) of 0.5-3.0mm. The parameters of the polyester corrugated conduits 10 in examples 1-12 are shown in Table 8 below.

The proximal conduit 2, the valve connecting conduit 3 and the distal conduit 4 are sequentially sewn to obtain an aortic valve preliminary conduit 1.

After the proximal conduit 2, the valve connecting conduit 3 and the distal conduit 4 are connected, the sewing operation such as folding at the joint is removed, and the related specifications of the three are as follows according to the structures shown in FIG. 1, FIG. 4, FIG. 7, FIG. 10 and FIG. 11:
In a natural state, an axial length of the proximal conduit 2 is not less than 5mm, and the proximal conduit includes at least two corrugations in an axial direction. The axial length of the valve connecting conduit 3 is 23-29mm, and the valve connecting conduit comprises 24-36 corrugations in a circumferential direction. In a natural state, the axial length of the distal conduit 4 is not less than 70mm, and the distal conduit includes at least 30 corrugations in an axial direction. The main specifications of the aortic valve preliminary conduit 1 in examples 1-12 are shown in Table 7 below.

**Table 7: Main specification difference of the aortic valve preliminary conduit 1 obtained in examples 1-12**

| Groups | Axial length of the proxim al conduit 2 (mm) | Complete corrugatio n number of the proximal conduit 2 | Axial length of the valve connecti ng conduit 3(mm) | Complete corrugatio n number of the valve connecting conduit 3 | Outer diameter of the joint between the two ends of the valve connectin g conduit 3 (mm) | Outer diameter of the maximum bulging portion of the valve connecting conduit 3 (mm) | Axial length of the distal conduit 4 (mm) | Complete corrugatio n number of the distal conduit 4 |
|---|---|---|---|---|---|---|---|---|
| Example 1 | 5.4±0.2 | 3 | 26.8±0.2 | 24 | 24.1±0.4 | 29.3±0.3 | 70.6±0.2 | 47 |
| Example 2 | 5.5±0.5 | 3 | 25.0±0.5 | 26 | 24.7±0.1 | 29.7±0.2 | 72.5±0.5 | 46 |
| Example 3 | 5.6±0.2 | 2 | 27.6±0.1 | 26 | 26.2±0.1 | 31.4±0.1 | 77.2±0.2 | 40 |
| Example 4 | 7.1±0.7 | 4 | 25.4±0.7 | 28 | 26.6±0.4 | 31.1±0.3 | 75.65±0. 7 | 45 |
| Example 5 | 6.2±0.5 | 5 | 26.8±0.5 | 28 | 28.3±0.2 | 33.4±0.2 | 78.4±0.5 | 63 |
| Example 6 | 5.5±0.2 | 3 | 25.5±0.2 | 30 | 28.5±0.2 | 33.9±0.1 | 73.75±0. 2 | 53 |
| Example 7 | 5.7±0.3 | 4 | 26.8±0.3 | 32 | 30.8±0.1 | 35.6±0.5 | 72.4±0.3 | 57 |
| Example 8 | 5.9±0.5 | 3 | 26.3±0.5 | 34 | 30.2±0.1 | 35.5±0.4 | 72.6±0.5 | 47 |
| Example 9 | 6.3±0.3 | 3 | 28.4±0.3 | 36 | 32.7±0.2 | 37.2±0.3 | 76.2±0.3 | 48 |
| Example 10 | 5.6±0.4 | 3 | 25.2±0.4 | 28 | 32.4±0.1 | 37.6±0.2 | 74.2±0.4 | 45 |
| Example 11 | 5.9±0.5 | 5 | 27.1±0.5 | 27 | 32.5±0.4 | 37.8±0.2 | 76±0.5 | 68 |
| Example 12 | 5.4±0.2 | 3 | 26.8±0.2 | 24 | 24.1±0.4 | 29.3±0.3 | 70.6±0.2 | 47 |

**Table 8: Parameters of the polyester corrugated conduit 10 in examples 1-12**

| Groups | Corresponding optimization experiment conduit | corrugated width (b in FIG. 2B) | corrugated height (a in FIG. 2B) | ratio of b/a |
|---|---|---|---|---|
| Example 1 | Experiment 12 | 1.48±0.02 | 1.13±0.02 | 1.31 |
| Example 2 | Experiment 13 | 1.56±0.01 | 1.26±0.04 | 1.24 |
| Example 3 | Experiment 15 | 1.89±0.16 | 1.44±0.04 | 1.31 |
| Example 4 | Experiment 18 | 1.65±0.03 | 1.25±0.01 | 1.32 |
| Example 5 | Experiment 19 | 1.24±0.02 | 0.93±0.04 | 1.33 |
| Example 6 | Experiment 27 | 1.38±0.02 | 1.03±0.01 | 1.34 |
| Example 7 | Experiment 28 | 1.27±0.02 | 0.94±0.01 | 1.35 |
| Example 8 | Experiment 33 | 1.54±0.04 | 1.13±0.03 | 1.36 |
| Example 9 | Experiment 54 | 1.58±0.01 | 1.16±0.03 | 1.36 |
| Example 10 | Experiment 58 | 1.62±0.01 | 1.15±0.06 | 1.41 |
| Example 11 | Experiment 59 | 1.11±0.01 | 0.84±0.05 | 1.32 |
| Example 12 | Experiment 12 | 1.48±0.02 | 1.13±0.02 | 1.31 |

It can be seen from Table 8 that the corrugated width (b in FIG. 2B) of the polyester corrugated conduit 10 of examples 1-12 is 1.11-1.89mm, the corrugated height (a in FIG. 2B) is 0.84-1.44mm, and the ratio of b/a is 1.24-1.41.

The valve connecting conduit 3 is in the shape of a regular lantern, or imitates the structure of the human physiological sinus, and the outer diameter of the valve connecting conduit 3 gradually increases from the joint of two ends thereof to the intermediate. Specifically, the outer diameter of the maximum bulging portion of the valve connecting conduit 3 ranges from 28-38mm, and the outer diameters of the joints at two ends of the valve connecting conduit 3 ranges from 24-33mm.

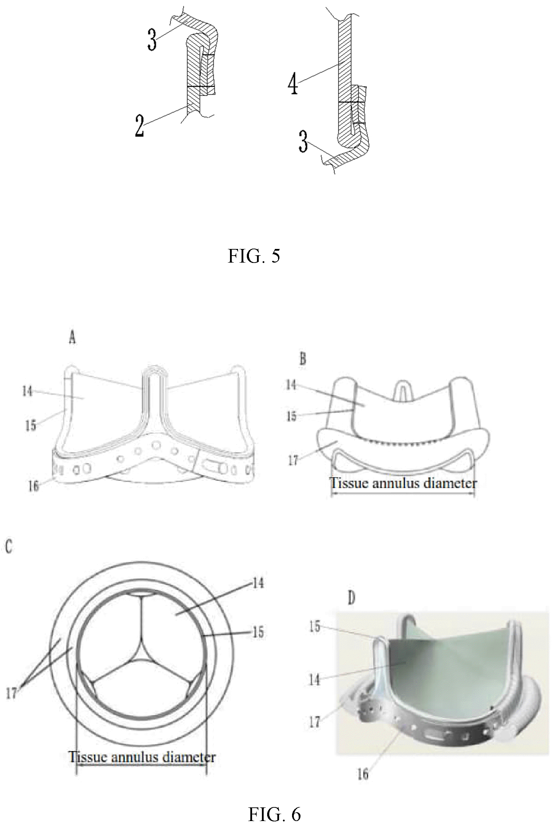

The sewing manner for the joint of the proximal conduit 2 and the valve connecting conduit 3 and the joint of the valve connecting conduit 3 and the distal conduit 4 can refer to FIG. 5, the joints of the proximal conduit 2/the distal conduit 4 and the valve connecting conduit 3 are folded outwards, the end portion of the valve connecting conduit 3 is respectively sleeved on the outer side of the proximal conduit 2/the distal conduit 4, and the suture passes back and forth through the inner and outer side walls of the joint of the valve connecting conduit 3 and the proximal conduit 2/the distal conduit 4. This sewing manner helps prevent bleeding at the joint of the valve connecting conduit 3 and the proximal conduit 2/the distal conduit 4, helps increase the durability of the conduit joint, and reduces the difficulty of manual sewing.

Collagen fiber coatings are attached to internal voids and internal and external surfaces of the aortic valve preliminary conduit 1 by adopting a preparation method of the collagen fiber coatings of 11 optimization experiments. The melting point 1 of the aortic valve preliminary conduit 1 obtained in examples 1-12 is the melting point of the collagen fiber subjected to the first chemical crosslinking (the collagen fiber subjected to the first chemical crosslinking is obtained by the "First: Experiments 1-60: Optimization of the preparation method of the conduit collagen fiber coating" ), and the melting point 2 is the melting point of the collagen fiber coating. Melting point 1 and melting point 2 were determined by DSC. For the preparation method of the related collagen fiber coating and the detection method of the melting point, refer to "First: Experiments 1-60: Optimization of the preparation method of the conduit collagen fiber coating". Specific melting point 1 and melting point 2 are shown in the following table:

**Table 9: Related melting points for examples 1-12**

| Groups | Preparation method of the corresponding optimization experiment | Melting Point 2(°C) | Melting Point 1(°C) | Difference between Melting Point 2 and Melting Point 1 (°C) |
|---|---|---|---|---|
| Example 1 | Experiment 12 | 76.93±0.02 | 68.54±0.13 | 8.39 |
| Example 2 | Experiment 13 | 79.84±0.03 | 68.54±0.13 | 11.30 |
| Example 3 | Experiment 15 | 78.08±0.02 | 68.54±0.13 | 9.54 |
| Example 4 | Experiment 18 | 77.59±0.01 | 69.82±0.10 | 7.77 |
| Example 5 | Experiment 19 | 79.26±0.04 | 69.82±0.10 | 9.44 |
| Example 6 | Experiment 27 | 77.75±0.02 | 70.36±0.09 | 7.39 |
| Example 7 | Experiment 28 | 78.80±0.03 | 70.36±0.09 | 8.44 |
| Example 8 | Experiment 33 | 78.25±0.05 | 70.61±0.02 | 7.64 |
| Example 9 | Experiment 54 | 78.94±0.06 | 71.24±0.03 | 7.70 |
| Example 10 | Experiment 58 | 80.90±0.02 | 71.36±0.02 | 9.54 |
| Example 11 | Experiment 59 | 80.97±0.01 | 71.36±0.02 | 10.31 |
| Example 12 | Experiment 12 | 76.93±0.02 | 68.54±0.13 | 8.39 |

In summary, the melting point 2 obtained in examples 1-12 was 76.93-80.97°C, the melting point 1 was 68.54-71.36°C, and the difference between the melting point 2 and the melting point 1 was 7.39-11.30°C.

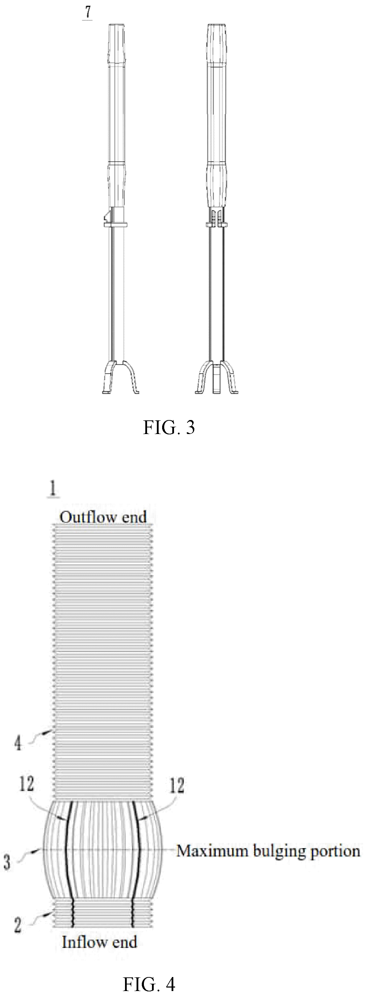

After coating, the limiting expandable biological valve 18 is connected to the limiting expandable aortic valve conduit to obtain the limiting expandable aortic valve conduit. Specifically, the limiting expandable aortic valve conduit further includes a valve holder 7 and a suture. The structure of the limiting expandable biological valve 18 can be found in CN116196150B, and the schematic diagram is shown in FIG. 6. The specific structure of the valve holder 7 is shown in FIG. 1 and FIG. 3. The limiting expandable biological valve 18 includes a valve leaflet 14, a valve frame 15, a valve seat 16 and a polyester sleeve with suture edge 17, and the valve leaflet 14 is made of bovine pericardium or porcine pericardium. The valve leaflet 14 and the valve frame 15 are connected, the valve seat 16 is sleeved with the polyester sleeve with suture edge 17, and then the two parts are connected through sutures to obtain the limiting expandable biological valve 18. After the limiting expandable biological valve 18 dysfunctions, since the valve seat 16 is expandable, it is possible to intervene a valve of the same size as the original prosthetic biological valve without opening the chest of the patient.

The suture is specifically a polyester suture in an amount of two to three. A polyester suture is used to connect the limiting expandable biological valve 18 with the valve holder 7. The rod body of the valve holder 7 is integrally connected with two symmetrically arranged knotting protrusions, a suture cutting groove 8 is formed between the two knotting protrusions, one end of the polyester suture is knotted at one knotting protrusion, is connected with the limiting expandable biological valve 18 downwards along the rod body of the valve holder 7, then crosses the suture cutting groove 8 upwards along the rod body of the valve holder 7, and then is knotted on the knotting protrusion. That is, after connecting with the limiting expandable biological valve 18, after crossing the suture cutting groove 8, knotting is performed on the knotting protrusion; during the operation, the suture is cut off at the suture cutting groove 8, so that the valve holder 7 and the limiting expandable biological valve 18 can be separated, this lead mode can facilitate the operation of the doctor, after the suture is cut off, the lengths of the free ends of the three sutures are not much different, when the doctor separates the valve holder 7 and the limiting expandable biological valve 18, the free ends of the three sutures have a separation time equivalent to that of the valve holder 7, the doctor does not need to wait for the separation of the valve holder 7 and the sutures in sequence, which facilitates the doctor's operation and saves surgical time.

The inflow end of the proximal conduit 2 is connected to the left ventricular outflow channel of the patient, and the polyester sleeve with suture edge 17 outside the valve seat 16 of the limiting expandable biological valve 18 is sewed on the inner surface of the joint of the valve connecting conduit 3 and the proximal conduit 2, or sewn onto the inner surface of the valve connecting conduit 3 (close to the inflow end of the valve connecting conduit 3). By optimizing the sewing manner of the joint of the valve connecting conduit 3 and the proximal conduit 2, the number of folds of the valve connecting conduit 3 and the bulging size of the valve connecting conduit 3, conditions can be provided for expanding the limiting expandable biological valve 18, the proximal conduit 2 is conveniently arranged so that a doctor can conveniently connect the proximal conduit 2 with the left ventricle outflow channel of the patient, and when the limiting expandable biological valve 18 is expanded, the inflow end of the proximal conduit 2 is connected with the left ventricle outflow channel of the patient, so that many factors at the left ventricle outflow channel do not need to be considered, and the problem of damaging the left ventricle outflow channel does not occur. In order to facilitate the surgical operation of the doctor, the proximal conduit 2, the valve connecting conduit 3 and/or the distal conduit 4 are provided with an identification line 12 along the axial direction thereof, and the identification line 12 is preferably provided on the proximal conduit 2, the valve connecting conduit 3 and the distal conduit 4, or may be provided only on the proximal conduit 2 and the valve connecting conduit 3, or may be provided only on the valve connecting conduit 3 and the distal conduit 4. The number of the identification lines 12 is preferably three, and the three identification lines 12 are respectively aligned with the positions of the protrusions (or referred to as the joints of the valve leaflet 14 and the valve leaflet 14) of the valve frame 15.

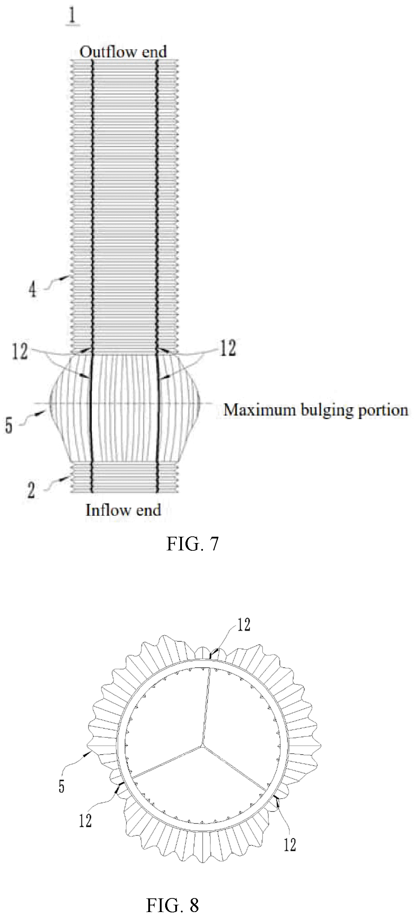

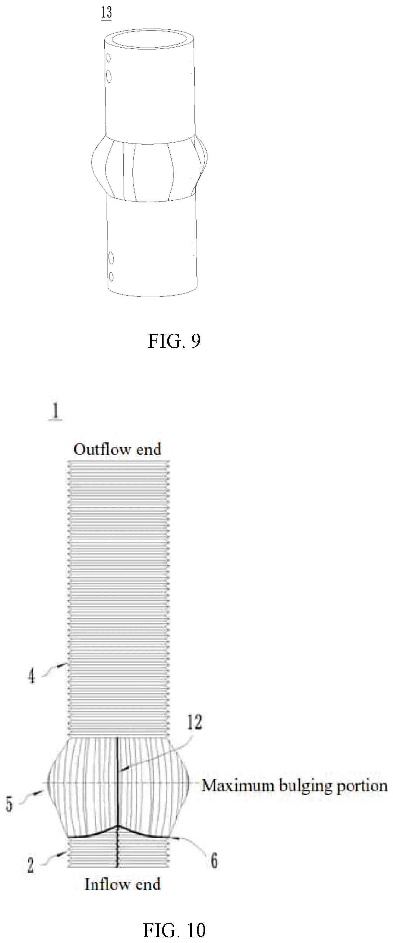

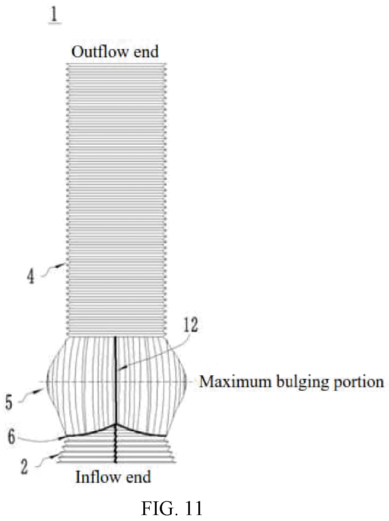

A specific structural schematic diagram of the aortic valve preliminary conduit 1 provided in examples 1-3 is shown in FIG. 4. A specific structural schematic diagram of the aortic valve preliminary conduit 1 provided in examples 4-7 is shown in FIG. 7. A specific structural schematic diagram of the aortic valve preliminary conduit 1 provided in examples 8-11 is shown in FIG. 10. A specific structural schematic diagram of the aortic valve preliminary conduit 1 provided in example 12 is shown in FIG. 11.

Specifically, in other preferred examples 4-7, the valve connecting conduit 3 includes a sinus structure, and "the valve connecting conduit 3" is named as "the expandable sinus connecting conduit 5", specifically, as shown in FIG. 7 to FIG. 8, three sinuses are uniformly distributed in the circumferential direction of the valve connecting conduit 3, and in a natural state, each sinus includes 8-12 corrugations. On one hand, the sinus structure can imitate a human physiological structure, and blood vortices are formed in each sinus, which is more convenient for opening and closing of the valve leaflet 14; on the other hand, the arrangement of the sinus structure can be more matched with the pre-expanded limiting expandable biological valve 18, and can also be matched with the post-expanded limiting expandable biological valve 18.

In other preferred examples 8-11, as shown in FIG. 10, the joint of the expandable sinus connecting conduit 5 and the proximal conduit 2 is a wavy wiring 6 having a direction consistent with the bottom of the limiting expandable biological valve 18. In addition to the corrugations on the corrugated conduit, the wavy wiring 6 can be sewn to the outflow end of the proximal conduit 2 (or the inflow end of the expandable sinus connecting conduit 5), and more space is reserved for the expanded biological valve seat 16.

In another preferred example 12, as shown in FIG. 11, compared with the limiting expandable aortic valve conduit provided by the above example 11, the longitudinal section of the proximal conduit 2 of the limiting expandable aortic valve conduit provided by the present example is trumpet-shaped, which is more convenient for the operation, the proximal conduit 2 is laid to the left ventricular outflow tract of the patient, which facilitates the suture of the doctor.

### 2: Preparation method of the limiting expandable aortic valve conduit of the examples 1-12

### Step 1: Cutting and connecting:

The polyester corrugated conduit 10 having an inner diameter of 20-22mm, an outer diameter of 23-25mm, a thickness of 0.15-0.45mm, a corrugated height (a in FIG. 2B) of 0.5-2.0mm, and a corrugated width (b in FIG. 2B) of 0.5-3.0mm were selected and cut in the axial direction to obtain a distal conduit 4, a valve intermediate conduit and a proximal conduit 2. The valve intermediate conduit is cut along its axial direction to obtain a rectangular corrugated conduit material, so that the corrugations are arranged along the axial direction of the whole limiting expandable aortic valve conduit, and then are sewn to form the valve connecting conduit 3. The size of the valve connecting conduit 3 needs to be matched with the pre-expanded limiting expandable biological valve 18 and the post-expanded limiting expandable biological valve 18. The aortic valve preliminary conduit 1 is obtained by sequentially connecting the proximal conduit 2, the valve connecting conduit 3 and the distal conduit 4.

In other preferred examples 4-7, the valve connecting conduit 3 is sleeved on the mold 13 (FIG. 9) for high-temperature setting to obtain the expandable sinus connecting conduit 5 with three sinuses uniformly distributed in the circumferential direction. The proximal conduit 2, the valve connecting conduit 3 and the distal conduit 4 are sewn in sequence to obtain an aortic valve preliminary conduit 1. Or, the proximal conduit 2, the valve connecting conduit 3 and the distal conduit 4 are first sewn in sequence to obtain the aortic valve preliminary conduit 1, and then the aortic valve preliminary conduit 1 is sleeved on the mold 13 for high-temperature shaping, so that the valve connecting conduit 3 becomes an expandable sinus connecting conduit 5 with three sinuses uniformly distributed in the circumferential direction.

In another preferred example 12, the valve intermediate conduit is cut along its axial direction to obtain a rectangular corrugated conduit, so that the corrugations are arranged along the axial direction of the whole limiting expandable aortic valve conduit, and then the valve connecting conduit 3 is sewn. The middle of the proximal conduit is cut along its axial direction, and then a trapezoidal corrugated conduit material is obtained by proper cutting, and the proximal conduit 2 with a trumpet-shaped longitudinal section is sewn.

Step 2: the collagen fiber coating is attached to the internal gaps and the inner and outer surfaces of the aortic valve preliminary conduit 1 by adopting the preparation method of the collagen fiber coating of 11 optimization experiments. The specific steps are as follows:
S1: Preparation of pig source type I collagen fibers
Refer to "First: Experiments 1-60: Optimization of the preparation method of the conduit collagen fiber coating" for preparation.
S2: First chemical crosslinking:
The obtained collagen fibers were added to glutaraldehyde aqueous solutions with different concentrations, mixed uniformly, stirred overnight at 30°C, centrifuged to collect precipitates, washed with pH7.0 phosphate buffer solution for 2-3 times to obtain collagen fibers subjected to the first chemical crosslinking. The melting point of the first chemically crosslinked collagen fiber (denoted "Melting Point 1") was determined by DSC. The main differences in S2 for examples 1-12 are as follows:

**Table 10: Differences in S2 for examples 1-12**

| Groups | Corresponding optimization Experiments | Concentration of glutaraldehyde aqueous solution | Melting Point 1 (°C) | Groups | Corresponding optimization Experiments | Concentration of glutaraldehyde aqueous solution | Melting Point 1 (°C) |
|---|---|---|---|---|---|---|---|
| Example 1 | Experiment 12 | 0.004% | 68.54±0.13 | Example 7 | Experiment 28 | 0.012% | 70.36±0.09 |
| Example 2 | Experiment 13 | 0.004% | 68.54±0.13 | Example 8 | Experiment 33 | 0.014% | 70.61±0.02 |
| Example 3 | Experiment 15 | 0.004% | 68.54±0.13 | Example 9 | Experiment 54 | 0.03% | 71.24±0.03 |
| Example 4 | Experiment 18 | 0.009% | 69.82±0.10 | Example 10 | Experiment 58 | 0.035% | 71.36±0.02 |
| Example 5 | Experiment 19 | 0.009% | 69.82±0.10 | Example 11 | Experiment 59 | 0.035% | 71.36±0.02 |
| Example 6 | Experiment 27 | 0.012% | 70.36±0.09 | Example 12 | Experiment 12 | 0.004% | 68.54±0.13 |

It can be seen from Table 10 that the concentration of glutaraldehyde aqueous solution in examples 1-12 was 0.004-0.035%, and the melting point 1 was 68.54-71.36°C.
S3: Preparation of coating
The collagen fibers subjected to the first chemical crosslinking obtained in step S2 are uniformly sprayed on the inner and outer surfaces of the aortic valve preliminary conduit 1 at a spraying density of 1.5-6mg/cm² (that is, 1.5-6mg is uniformly sprayed on the inner and outer surfaces of the conduit per square centimeter), and after spraying, drying is performed in an oven at 31-33°C to obtain the aortic valve preliminary conduit 1 adsorbed with the collagen fibers. The main differences in S3 for examples 1-12 are as follows:

**Table 11: Differences in S3 for examples 1-12**

| Groups | Spraying density in S3 (mg/cm²) | Drying temper ature in S3 | Groups | Spraying density in S3 (mg/cm²) | Drying tempera ture in S3 | Groups | Spraying density in S3 (mg/cm²) | Drying temperat ure in S3 |
|---|---|---|---|---|---|---|---|---|
| Example 1 | 1.5 | 31 | Example 5 | 4.5 | 33 | Example 9 | 4 | 33 |
| Example 2 | 2.5 | 32 | Example 6 | 3 | 31 | Example 10 | 3.5 | 32 |
| Example 3 | 5.5 | 32 | Example 7 | 3 | 32 | Example 11 | 4.5 | 33 |
| Example 4 | 2.5 | 32 | Example 8 | 3.5 | 32 | Example 12 | 1.5 | 31 |

S4: Fully immersing the aortic valve preliminary conduit 1 adsorbed with collagen fibers in a second crosslinking agent solution, and standing for 20 minutes at room temperature to obtain the aortic valve preliminary conduit 1 immersed with the crosslinking agent adsorbed with collagen fibers.

The preparation method of the second crosslinking agent solution comprises the following steps: weighing N-hydroxysuccinimide (NHS) and 1-(3-dimethylaminopropyl)-3-ethylcarbodiimide hydrochloride (EDC) according to a mass ratio of 1:1 in a centrifuge conduit, and adding a pH7.2 phosphate (PBS) solution to obtain the crosslinking agent solution, wherein the concentrations of NHS and EDC are 27-40mg/mL.

**Table 12: Differences in S4 for examples 1-12**

| Groups | Concentration of crosslinking agent in S4 (mg/mL) | Groups | Concentration of crosslinking agent in S4 (mg/mL) | Groups | Concentration of crosslinking agent in S4 (mg/mL) |
|---|---|---|---|---|---|
| Example 1 | 27 | Example 5 | 37 | Example 9 | 36 |
| Example 2 | 38 | Example 6 | 30 | Example 10 | 39 |
| Example 3 | 31 | Example 7 | 35 | Example 11 | 40 |
| Example 4 | 29 | Example 8 | 33 | Example 12 | 27 |

### S5: Second chemical crosslinking

The first chemically crosslinked collagen fiber obtained from S2 was formulated into a collagen fiber solution with a concentration of 3.5-5.5mg/mL using pH7.2 phosphate (PBS) solution. The aortic valve preliminary conduit 1 immersed with the crosslinking agent and adsorbed with the collagen fibers is immersed in the collagen fiber solution, and is allowed to stand at room temperature for 2 hours or more (preferably, stand overnight), and is fully dried at 31-35°C, at this time, the collagen fibers subjected to twice chemical crosslinking form a collagen fiber coating, which exists in the internal gaps of the aortic valve preliminary conduit 1 and the internal and external surfaces of the conduit, to obtain the aortic valve preliminary conduit 1 attached with the collagen fiber coating. The melting point of the collagen fiber coating (denoted as "melting point 2") was determined by differential scanning calorimetry DSC.

**Table 13: Differences in S5 for examples 1-12**

| Groups | Concentrat ion of collagen fiber solution in S5 (mg/mL) | Drying tempera ture in S5 (°C) | Melting Point 2 (°C) | Groups | Concentrat ion of collagen fiber solution in S5 (mg/mL) | Drying tempera ture in S5 (°C) | Melting Point 2 (°C) |
|---|---|---|---|---|---|---|---|
| Example 1 | 3.5 | 31 | 76.93±0.02 | Example 7 | 4.5 | 32 | 78.80±0.03 |
| Example 2 | 5.5 | 32 | 79.84±0.03 | Example 8 | 4.5 | 32 | 78.25±0.05 |
| Example 3 | 4 | 35 | 78.08±0.02 | Example 9 | 5 | 33 | 78.94±0.06 |
| Example 4 | 3.5 | 32 | 77.59±0.01 | Example 10 | 5.5 | 32 | 80.90±0.02 |
| Example 5 | 5 | 33 | 79.26±0.04 | Example 11 | 5.5 | 33 | 80.97±0.01 |
| Example 6 | 4 | 31 | 77.75±0.02 | Example 12 | 3.5 | 31 | 76.93±0.02 |

### Step 3: Drying

The limiting expandable biological valve 18 is immersed in 100% glycerol, and water molecules and glycerol are fully replaced to obtain a dried limiting expandable biological valve 18. As shown in FIG. 6, the tissue annulus diameter range of the limiting expandable biological valve 18 is 19.0-29.0mm, the expandable diameter range is 2-3mm, and the expanded tissue annulus diameter range is 21.0-31.0mm.

### Step 4: Assemblying

One end of the valve holder 7 and the limiting expandable biological valve 18 are connected through sutures, and then the limiting expandable biological valve 18 and the aortic valve preliminary conduit 1 obtained in the third step are connected through sutures to obtain a limiting expandable aortic valve conduit.

### Third: Relevant testing of examples 1-12

### 1: Physical properties

Visual observation shows that the limiting expandable aortic valve conduit obtained in examples 1-12 has symmetrical valve leaflets, neat alignment, no wrinkles and scratches; the suture connecting pins are tidy and uniform, free of cracks and exposed to the wireless head; the joint of the valve conduit, the free end, the wireless head and the knot are exposed, free of burrs, broken wires or drawn wires; and the conduit has a wrinkled structure and has elasticity and stretchable. The thickness of the limiting expandable aortic valve conduit obtained in examples 1-12 was 0.33±0.08mm. The valve of the limiting expandable aortic valve conduit can be expanded through balloon assisted expanding, can be completely expanded under the condition of 607.9±101.3kPa (6±1atm), and has no damage after expanding.

### 2: Chemical properties

Prepare the extraction of the test solution according to GB/T 14233.1, specifically, add water at a ratio of 0.2g sample to 1mL, extract for 72 hours at 37±1°C, separate the sample from the liquid, cool to room temperature, and use as the test solution. Detect heavy metal content according to methods 5.6.1 and 5.9.2 in GB/T 14233.1-2022. Experimental results show that the total content of barium, chromium, copper, lead and tin in the test solution of the limiting expandable aortic valve conduit obtained in examples 1-12 does not exceed 1µg/mL, the content of cadmium does not exceed 0.1µg/mL, and the heavy metal content (calculated as lead) does not exceed 1mg/L.

The ethylene oxide residue of the limiting expandable aortic valve conduit obtained in examples 1-12 was detected according to GB/T 14233.1-2022 method, and after measurement, the ethylene oxide residue of the limiting expandable aortic valve conduit obtained in examples 1-12 did not exceed 10µg/g.

### 3: Mechanical experiments

Referring to YY_T 0500-2021, specifically, two circular pins are placed in the limiting expandable aortic valve conduit, and the limiting expandable aortic valve conduit is stretched in the circumferential direction at a stable rate of 100mm/min until the limiting expandable aortic valve conduit is broken to obtain an inner diameter of the limiting expandable aortic valve conduit during breaking, and the maximum circumferential tensile elongation is obtained by (the inner diameter length of the limiting expandable aortic valve conduit during breaking-the original inner diameter length of the limiting expandable aortic valve conduit)/the original inner diameter length of the limiting expandable aortic valve conduit × 100%. The elastic deformation rate is measured using conventional measurement methods in the art. Specifically, the elastic deformation rate is calculated based on the elastic deformation section of the tensile curve, wherein the tensile curve is drawn when the maximum circumferential tensile elongation is measured.

**Table 15: Related mechanical parameters of the limiting expandable aortic valve conduit's distal conduit of examples 1-12**

| Groups | Maximum circumferential tensile elongation | Elastic deformation rate | Value of elastic deformation rate as a percentage of the maximum circumferential tensile elongation | Maximum tensile force |
|---|---|---|---|---|
| Example 1 | 3.6±0.2 | 2.1±0.2 | 58% | 25.3±0.2 |
| Example 2 | 4.2±0.5 | 1.8±0.3 | 43% | 34.7±0.1 |
| Example 3 | 2.9±0.3 | 1.1±0.2 | 38% | 16.2±0.1 |
| Example 4 | 4.8±0.1 | 1.0±0.3 | 21% | 45.1±0.1 |
| Example 5 | 2.6±0.2 | 1.2±0.1 | 46% | 18.8±0.2 |
| Example 6 | 2.2±0.1 | 1.4±0.2 | 64% | 21.4±0.1 |
| Example 7 | 2.3±0.2 | 1.2±0.4 | 52% | 55.9±0.1 |
| Example 8 | 2.4±0.1 | 1.4±0.1 | 58% | 17.4±0.1 |
| Example 9 | 2.2±0.1 | 1.5±0.4 | 68% | 25.7±0.2 |
| Example 10 | 2.3±0.2 | 1.3±0.5 | 57% | 13.5±0.1 |
| Example 11 | 2.4±0.3 | 1.8±0.1 | 75% | 18.2±0.1 |
| Example 12 | 3.5±0.3 | 2.3±0.1 | 66% | 25.5±0.3 |

In summary, the maximum circumferential tensile elongation range of the limiting expandable aortic valve conduit's distal conduit in examples 1-12 is 2.2-4.8%, the elastic deformation rate range of the distal conduit is 1.0-2.3%, the value range of the elastic deformation rate in the maximum circumferential tensile elongation is 21-75%, and the maximum tensile force range of the distal conduit is 13.5-55.9N. The mechanical properties of the proximal conduit and distal conduit are consistent.

### 4: Total Water Permeability Experiment

The flow rate of water leakage through the conduit wall at 16kPa (120mmHg) pressure was measured as performed at YYT0500-2021. The specific test steps are:
Prior to testing, the limiting expandable aortic valve tubing was specifically wetted with clean filtered water at room temperature (or at a specified temperature). Experiments were conducted on the limiting expandable aortic valve conduit in an implantable state. The distal end of the expandable aortic valve conduit is sealed using a plug or adapter adapted to the inner diameter of the conduit. The proximal end of the limiting expandable aortic valve conduit is connected to an adapter with a specific inner diameter to ensure sealing. The adapter and limiting expandable aortic valve conduit are connected to a pressure generating and measuring fixture. By gradually increasing the intraluminal pressure of the limiting expandable aortic valve conduit, the trapped air is discharged. The pressure was brought to 16.0kPa±0.3kPa (120mmHg±2mmHg), the pressure or flow was maintained stable, and the amount of water seeping through the walls within 60 seconds was measured and the total water permeability was calculated. Total water permeability is expressed as ml/(cm²·min).

**Table 14: Total water permeability of the limiting expandable aortic valve conduit obtained in examples 1-12**

| Groups | Total Water Permeability (ml/(cm²·min)) | Groups | Total Water Permeability (ml/(cm²·min)) |
|---|---|---|---|
| Example 1 | 2.24±0.09 | Example 7 | 0.26±0.07 |
| Example 2 | 0.18±0.03 | Example 8 | 0.42±0.21 |
| Example 3 | 0.35±0.05 | Example 9 | 0.65±0.23 |
| Example 4 | 0.97±0.21 | Example 10 | 3.67±0.01 |
| Example 5 | 0.12±0.04 | Example 11 | 1.22±0.08 |
| Example 6 | 0.34±0.01 | Example 12 | 2.28±0.03 |

Since the total water permeability of the conduits obtained by 11 optimization experiments is relatively small, in the examples 1-12, the limiting expandable aortic valve conduits of examples 1 and 10-12 have a large total water permeability, and the main water permeability part of the limiting expandable aortic valve conduit is mainly at the joint between the valve connecting conduit 3 and the distal conduit 4, and the joint between the proximal conduit 2 and the valve connecting conduit 3.

It can be seen from Table 14 that the limiting expandable aortic valve conduit obtained in examples 2-9 has a small total water permeability, which indicates a good anti-bleeding effect. By comparison, it can be found that the limiting expandable aortic valve conduit obtained in examples 2-9 has a melting point 2 of 77.59-79.84°C, a melting point 1 of 68.54-71.24°C, a difference between the melting point 2 and the melting point 1 of 7.39-11.30°C, a corrugated width (b in FIG. 2B) of 1.24-1.89mm, a corrugated height (a in FIG. 2B) of 0.93-0.93mm, and a ratio of b/a (a ratio of the corrugated width (b in FIG. 2B) to the corrugated height (a in FIG. 2B)) of 1.24-0.93mm.

### Fourth: Animal experiments

Sheep have similar hemodynamic characteristics and laboratory indicators to humans, particularly blood coagulation system characteristics and human proximity, often selected as animal models for heart valve replacement and vascular replacement. Sheep have a gentle temperament, are easy to manage, are less prone to infection after surgery, are easier to control after long-term feeding, and have a high long-term survival rate. Therefore, sheep were chosen as experimental animals in this experiment.

Sheep were selected as experimental animals. Bentall surgery was performed on experimental animals to evaluate the safety and effectiveness of the limiting expandable aortic valve conduit in animals, and to evaluate the feasibility and safety of placing the interventional valve-in-valve in the limiting expandable biological valve after balloon expanded. After limiting expanded, a valve-in-valve is implanted to simulate the requirement of the future interventional valve-in-valve, an animal experiment evaluates the heart function of an animal after subsequent intervention, monitors blood routine, and assists in evaluating the safety and effectiveness of the limiting expandable aortic valve conduit.

### 1: Inspection criteria and feeding

Experimental animals were purchased from Xi'an Dilep Biomedical Co., Ltd. This experiment used sheep, male, with an average preoperative weight of 56.35±6.911 kg and a weight range of 42kg-75kg; the age of 12 months or above; normal temperaturel, no symptoms such as cold, fever, cough etc., passing the quarantine inspection, and undergoing blood testing during quarantine; the observation period is 7 days, and after confirmation of qualification, it will be numbered; the preoperative fasting time is 12-16 hours, and free to drink water; if the animal coagulation mechanism is abnormal, it cannot be selected; and if there are other animals which may affect the experiment result, selection is not carried out. The experimental animals were adapted to the environment and isolated quarantine one week before surgery, and the postoperative animals were raised to animal rooms. The facility temperature was in the range of 16-26°C, and each animal was fed in a single cage. The sheep monitoring cage was cleaned 2 times per day. The feed is fed with standardized feed, is free to eat, and is free to drink water.

### 2: Grouping and quantity

Experimental animals had a total of 24 sheep. Experiments were performed using the limiting expandable aortic valve conduit obtained in eight examples, which are respectively examples 2-9, and three sheep were used for each example.

30 days after Bentall surgery using the limiting expandable aortic valve, eight sheep (one for each of the eight examples) reached the end point of the experiment, and the rest 16 sheep were used to balloon limited dilation of the implanted limiting expandable biological valve through a minimally invasive thoracotomy, and preoperative ultrasound, dilation pressure, and DSA measurements of the expanded valve size were recorded to confirm the limited position. Then, the corresponding type of interventional valve is inserted through a conduit into the expanded valve to verify the reliability of the valve seat and the presence of perivalvular leakage. After surgery, the animals will continue to be raised for 30 days until the end of the experiment, and undergo ultrasound, dissection, and other examinations. During the experiment, animal information was recorded, including body weight, surgical time, survival days, and the limiting expandable aortic valve conduit information and the like. Before surgery, measure blood routine, blood biochemistry, coagulation function, blood gas. After surgery, measure blood routine, blood biochemistry, coagulation function, and blood gas.

### 3: Experimental protocol

Preoperative preparation: preoperative adaptive feeding for one week: 12 hours of light dark cycle, room temperature 22±6°C, humidity 40-70%, preoperative fasting time 12-16 hours, and free to drink water. The aortic annulus diameter of each animal was measured using CT before the surgery started. Scopolamine hydrobromide injection (dose 0.01mg/kg, intramuscular injection) reduces respiratory secretions, inhale isoflurane through a face mask, and insert isoflurane through tracheal intubation. A venous pathway is established, an electrocardiograph monitor is connected, and heart rate, blood pressure and blood oxygen saturation are monitored.

The 24 experimental animals were subjected to a Bentall surgery according to a conventional surgery, and the specific surgery was as follows:
(a) Performing transthoracic Doppler color ultrasound examination according to the requirements of routine examination, and recording preoperative data; (b) lying the animal on the right side, performing conventional disinfection on the chest, performing sterile spreading, performing chest opening on the left side, performing whole body heparinization after taking a fourth rib, and exposing the heart; (c) establishing extracorporeal circulation, placing a left heart drainage conduit, blocking the ascending aorta, perfusing cold blood arrest fluid through the aorta and (or) left and right coronary arteries, and cooling the surface of the heart with ice debris; (d) performing oblique incision on the ascending aorta, cutting the ascending aorta and cutting diseased valve leaflets; (e) performing proximal anastomosis to limit replacement of the root of the expandable aortic valve conduit, and performing continuous or intermittent suturing; (f) punching corresponding left and right coronary artery opening positions of a valve connecting conduit of the limiting the expandable aortic valve conduit, directly sewing button shaped left and right coronary artery openings on the valve connecting conduit, and performing continuous sewing the distal end of the limiting expandable aortic valve conduit and the ascending aorta; (g) performing ultrasonic examination on the function of the artificial biological valve and the hemodynamic conditions through the esophagus; (h) after no active hemorrhage in the thoracic cavity is examined, placing the thoracic cavity drainage conduit, intermittently closing the thoracic cavity layer by layer, placing the thoracic cavity drainage conduit to be connected with the drainage bottle; (i) pulling out the cervical vein and the femoral artery puncture sheath, and pressing for at least 10 minutes; and (j) after the experimental animals have recovered their spontaneous breathing and corneal radiation, and their blood pressure and heart rhythm have stabilized, remove the endotracheal tube and observe in a natural position for 30 minutes.

30 days after Bentall surgery, 13 experimental animals were subjected to balloon dilation through minimally invasive thoracotomy, and the specific surgery was as follows:
(a) Animals should be fasted for 12-16 hours before surgery and is free to drink water. Preoperative blood sampling and laboratory testing are used as baseline values. Blood was drawn intravenously for testing before animal surgery. (b) Scopolamine hydrobromide injection (dose 0.03mg, intramuscular injection) reduces respiratory tract secretions, inhale isoflurane through a face mask, and insert isoflurane through tracheal intubation. A venous pathway is established. (c) Performing arterial puncture to perform invasive blood pressure monitoring on experimental animals. The electrode is connected to observe whether the electrocardiogram is normal. (d) The medication administration during the preparation process is determined by the doctor. Maintain isoflurane respiratory anesthesia and monitor the anesthesia conditions by observing the animal's blood pressure, whether the ears are moving, whether the chin is open or closed, eye reflexes, and whether the toes are clamped. Administer ceftriaxone sodium before the surgery begins, and if the surgery time exceeds 5 hours, administer ceftriaxone sodium again. (e) perform transthoracic Doppler color ultrasound examination according to the requirements of routine examination, and recording preoperative data; (f) the animal lying on the right side, small incision on the left chest, routine disinfection of the skin in the chest and left inguinal area, puncture of the left femoral vein, insertion of a sheath to establish a contrast imaging pathway; (g) performing cardiac color ultrasound on the chest or the heart surface according to the requirements of conventional examination, and recording data; (h) cutting the skin, subcutaneous tissue and muscle layer by layer, carefully cutting the sternum with scissors, cutting the pericardium, suspending the heart with a 6 × 14 wire pericardium basket, and selecting the specification of the corresponding interventional bovine pericardium valve in combination with the specification of the implanted M23 bovine pericardium valve model. Expose the apex of the heart and suture the purse with 5-0 prolene suture at the apex of the heart; (i) penetrating the puncture sheath into the left chamber from the center of the purse bag to the direction of the left chamber outflow tract, placing a medical guide wire along the puncture sheath core, confirming that the medical guide wire enters the left atrium, and withdrawing the puncture sheath; (j) inserting a 25 gauge balloon along the medical guide wire and use DSA imaging to guide the balloon into the aortic valve position. Use a pressure pump to inflate the balloon and maintain it at 5.0-6.0 atm for about 2 seconds to complete the expansion of the limiting expandable biological valve 18; (h) along the medical guide wire, the delivery sheath pre-installed with the interventional valve in the middle of the 24F valve is sent into the mitral valve, after confirming the appropriate position of the stent through DSA imaging, the valve stent is expanded with a balloon pressure pump, and after the valve is completely expanded, the balloon is retracted, the delivery sheath is withdrawn, and the guide sheath and medical guide wire are withdrawn. After the purse is tightened, the apical wound has no bleeding, and the purse is ligated. (i) Ultrasound examination of the function of the interventional bovine pericardial valve, as well as hemodynamic conditions, was performed, and after checking for no active bleeding in the chest cavity, the chest was gradually closed layer by layer. After the sheep's spontaneous breathing, corneal radiation recovery, and stable blood pressure and heart rhythm, remove the endotracheal tube and observe in a natural position for 30 minutes.

Postoperative treatment: After the operation is successful, the experimental animals are returned back to the animal room to continue observation and feeding. Food and water are provided periodically. After chest closing, bupivacaine hydrochloride injection was used as intercostal sealing to relieve pain. Animal conditions should be closely monitored throughout post-operative care. Within 7 days after surgery, cefotaxime sodium was injected intramuscularly and recorded twice daily. The heparin sodium injection was administered intravenously at a dose of 1.5mg/kg or 100-200U/kg during the operation, and maintain ACT > 500s. On the day after surgery, warfarin sodium tablets were given aspirin with an initial dose of 5mg + 100mg, the dose of warfarin sodium tablets was adjusted based on the INR results to maintain the INR value within the range of 1.5-2.5. When bleeding and other conditions occur, the anticoagulation scheme is adjusted according to doctor requirements. When bleeding and other situations occur, the anticoagulation scheme is adjusted according to doctor requirements.

Experimental endpoint: all animals reaching and not reaching the experimental endpoint were subjected to general anatomical and pathological examination in this experiment. After the endpoint animals are euthanized in the anesthesia state, the heart and other main organs are taken out and subjected to organ dissection, including brain, kidney, spleen, lung and liver, and the appearance, profile examination and thrombosis conditions of each organ are observed by naked eyes: the heart is cut, the thrombosis conditions of the valve inflow and outflow surfaces are observed, and photographing and archiving are performed. Main organs such as heart, brain, kidney, spleen, lung, liver and the like are made into sections, and thrombus examination, valve change condition and secondary or other pathological examination of each organ are performed through the sections.

### 4: Observation index

General conditions include the condition of anastomosis hemorrhage, as well as the feeding, mental state, living habit, urination, shortness of breath, oliguria, behavioral abnormalities, etc. of experimental animals. Blood examination: including blood gas, blood routine (including plasma free hemoglobin, white blood cells, neutrophils, lymphocyte number, monocyte number, eosinophils, basophils, erythrocyte number, hemoglobin, erythrocyte accumulation, platelet number), blood biochemistry (including total bilirubin, direct bilirubin, alanine aminotransferase, aspartate aminotransferase, C-reactive protein, alkaline phosphatase, glutaminotransferase, total protein, albumin, creatinine, uric acid, urea nitrogen), blood coagulation (including prothrombin time, thrombin time, activated partial coagulase time), immunology and the like; and evaluating the reactions of animal blood hemolysis, inflammation, immunity and the like after implantation. Transthoracic ultrasonic examination: observing the presence or absence of neoplasm at the valve and annulus, abnormal blood flow in the conduit and the like; measuring the volume of the left chamber, the blood flow velocity and pressure difference of the aortic valve and the ejection fraction of the left chamber; and observing and recording the size of each heart cavity of the experimental animal, the regurgitation degree of each valve and regurgitation. General anatomical and pathological inspection: all animals in this experiment that reached and did not reach the end of the experiment were subjected to general anatomical and Pathological Inspection. General anesthesia, femoral vein puncture and blood drawing (blood routine, liver and kidney function); whole body heparinization, 3mg/kg (the purpose of heparinization is to prevent blood coagulation after animal death, which is confused with thrombus formed in the limiting expandable biological valve); the animal is killed by bleeding under whole hemp; the conditions of tissue around the limiting expandable biological valve and the tissue joint in the implanted animal are observed, and no morphological changes, adhesions, ibrocystic cavities and the like exist, and photographing recording is carried out; the valve leaflet morphology change, thrombus formation conditiosn and calcification conditions of the limiting expandable biological valve are observed by naked eyes; if thrombus exists, the weight (wet weight) of the thrombus and the surface proportion of the implant are recorded; the shape of the limiting expandable aortic valve conduit of the prosthesis is observed, and the deformation status is recorded; the limiting expandable biological valve and the surrounding tissue are left to be made into pathological sections, and pathological change is observed under a lens: the limiting expandable biological valve and the surrounding 3mm area are sampled, fixed in 10% neutral formalin for 10-14 days, paraffin embedding, tissue sections and HE staining; organ dissection is carried out: the heart, the lung, the spleen, the liver, the kidney and the brain are dissected, general examination is carried out, and the condition of an incision of an operation (chest) is observed, and no infarction or abnormal lesions are photographed and recorded. Processing and analysis of the heart specimen: the left atrium was cut, and the left ventricle was observed from the endocardial surface at the valve placement position. Subsequently, the ventricular cavity wall and the peripheral tissue of the aortic valve are taken, a specimen is preserved in a fresh or 4°C refrigerator, and the specimen is delivered and examined within 24 hours of day death or accidental death at the end point of the experiment. Whole organ examination focuses on the valve surface changes and the thromboembolism, infection and necrosis of each organ, and conventional optical lenses take materials, wherein the materials include the left ventricle, the left atrium, the limiting expandable aortic valve conduit and the peripheral tissue of the aortic valve, and include the limiting expandable biological valve.

### 5: Animal experimental results

(1) In the eight examples, during Bentall surgery using the limiting expandable aortic valve conduit of the example 4, a relatively serious bleeding occurs at the joint of the proximal duct and the left ventricular outflow duct, and it is assumed that the elastic deformation rate accounts for a relatively low value of the maximum circumferential tensile elongation, so the elastic deformation rate accounts for a value of the maximum circumferential tensile elongation should be greater than 21%, which may be controlled at 38-68%.
(2) In the eight Examples, the limiting expandable aortic valve conduit obtained in examples 2-3 and 5-9 has no uncontrolled anastomosis bleeding for animals, intraoperative and postoperative surgeries. Moreover, the conduit has strong shear resistance, no kinking phenomenon and excellent mechanical properties.

After minimally invasive intervention, the experimental animals using the limiting expandable aortic valve conduits obtained in examples 2-3 and 5-9 generally had good conditions during survival and feeding, normal body temperature, diet and excretion, good autonomous activity, no abnormal behavior manifestation such as shortness of breath, oliguria, significant weight loss, fever, anorexia, and mania, and successfully survived to the end point, and no complications such as myocardial infarction, severe arrhythmia, embolization, and prosthetic valve dysfunction. Blood routine, blood biochemistry and blood coagulation are not abnormal. The electrolyte at each follow-up period after the extracardiac radiofrequency ablation operation is examined, the liver and kidney function values are almost within the normal range, and the preoperative and follow-up periods of the coagulation INR values are about 1.0. The total blood cell number, morphology and quality are not abnormal; detection results such as white blood cells, red blood cells, hemoglobin and platelets show that the overall result is stable, and important blood routine indexes are not found to be abnormal. No liver and kidney dysfunction was observed.

Obviously, those skilled in the art can make various changes and modifications to the present invention without departing from the spirit and scope of the present invention. The present disclosure is not limited to the precise structure already described above and shown in the accompanying drawings, and various modifications and changes may be made without departing from the scope thereof. The scope of the present disclosure is limited only by the appended claims. Thus, the present invention is also intended to embrace such modifications and variations provided they fall within the scope of the claims and their equivalents.

## Claims

1. A limiting expandable aortic valve conduit, **characterized in that**, comprising an aortic valve preliminary conduit attached with a collagen fiber coating, wherein the aortic valve preliminary conduit comprises a proximal conduit, a valve connecting conduit and a distal conduit;
the collagen fiber coating is formed by performing second chemical crosslinking on collagen fibers which have been subjected to first chemical crosslinking to have a melting point of 68.54-71.24°C; the collagen fiber coating is attached to internal gaps and surfaces of the aortic valve preliminary conduit; the melting point of the collagen fiber coating is 77.59-79.84°C, and the difference between the melting point of the collagen fiber coating and the melting point of the collagen fibers subjected to first chemical crosslinking ranges from 7.39-11.30°C.

2. The limiting expandable aortic valve conduit according to claim 1, **characterized in that**, in a natural state of the aortic valve preliminary conduit, an axial length of the proximal conduit is not less than 5mm, and the proximal conduit comprises at least 2 corrugations in an axial direction; an axial length of the valve connecting conduit is 23-29mm, and the valve connecting conduit comprises 24-36 corrugations in a circumferential direction; an axial length of the distal conduit is not less than 70mm, and the distal conduit comprises at least 30 corrugations in an axial direction.

3. The limiting expandable aortic valve conduit according to claim 2, **characterized in that**, the proximal conduit, the valve connecting conduit and the distal conduit are made of polyester corrugated conduits of a same specification, and a ratio of a corrugated width to a corrugated height of the polyester corrugated conduits is 1.24-1.36.

4. The limiting expandable aortic valve conduit according to claim 1, **characterized in that**, a maximum circumferential tensile elongation range of the distal conduit is 2.2-4.8%, an elastic deformation rate range of the distal conduit is 1.0-2.3%, a value of the elastic deformation rate in the maximum circumferential tensile elongation range is 38-68%, and a maximum tensile force range of the distal conduit is 13.5-55.9N.

5. The limiting expandable aortic valve conduit according to claim 1, **characterized in that**, an outer diameter of a maximum bulging portion of the valve connecting conduit ranges from 28-38mm, and an outer diameter of a joint at two ends of the valve connecting conduit ranges from 24-33mm.

6. The limiting expandable aortic valve conduit according to claim 1, **characterized in that**, further comprising a limiting expandable biological valve, wherein the limiting expandable biological valve is sewn on an inner surface of the valve connecting conduit, or the limiting expandable biological valve is sewn on an inner surface of a joint of the valve connecting conduit and the proximal conduit.

7. The limiting expandable aortic valve conduit of claim 6, **characterized in that**, further comprising a valve holder connected to the limiting expandable biological valve.

8. The limiting expandable aortic valve conduit according to claim 7, **characterized in that**, two symmetrically arranged knotting protrusions are integrally connected to the rod body of the valve holder, and a suture cutting groove is formed between the two knotting protrusions.

9. The limiting expandable aortic valve conduit according to claim 1, **characterized in that**, a joint of the proximal conduit/distal conduit and the valve connecting conduit is folded outward, an end portion of the valve connecting conduit is respectively sleeved on an outer side of the proximal conduit/distal conduit, and sutures pass back and forth through inner and outer side walls of the joint of the valve connecting conduit and the proximal conduit/distal conduit.

10. A method for preparing a limiting expandable aortic valve conduit, **characterized in that**, comprising:
Step 1: cutting and connecting:
selecting a polyester corrugated conduit with a corrugated width of 1.24-1.89mm, a corrugated height of 0.93-1.44mm, and a ratio of the corrugated width to the corrugated height of 1.24-1.36, cutting the polyester corrugated conduit in an axial direction of the polyester corrugated conduit, and cutting the polyester corrugated conduit to obtain a distal conduit, a valve intermediate conduit and a proximal conduit; and cutting the valve intermediate conduit along the axial direction of the valve intermediate conduit to obtain a rectangular corrugated conduit material, arranging the corrugations along the axial direction of the whole limiting expandable aortic valve conduit, and then sewing into a valve connecting conduit;
obtaining an aortic valve preliminary conduit by sequentially connecting a proximal conduit, a valve connecting conduit and a distal conduit with sutures;
Step 2: attaching a collagen fiber coating on the internal gaps and the inner and outer surfaces of the aortic valve preliminary conduit:
S1: preparing collagen fibers;
S2: obtaining a collagen fiber having a melting point of 68.54-71.24°C through the first chemical crosslinking by using biologically derived collagen as raw material and glutaraldehyde as crosslinking agent;
S3: spraying the collagen fiber subjected to the first chemical crosslinking obtained in step S2 on the polyester corrugated conduit, and drying to obtain the polyester corrugated conduit adsorbed with the collagen fiber, wherein the spraying density of the collagen fiber subjected to the first chemical crosslinking on the surface of the polyester corrugated conduit is 1.5-6mg/cm²;
S4: immersing the polyester corrugated conduit adsorbed with the collagen fiber in a crosslinking agent solution to obtain the polyester corrugated conduit immersed with the crosslinking agent adsorbed with the collagen fiber, wherein the crosslinking agent solution is a compound solution of N-hydroxysuccinimide and 1-(3-dimethylaminopropyl)-3-ethylcarbodiimide hydrochloride, the compound solution is compounded according to a mass ratio of 1:1, and the concentration is 27-40mg/mL;
S5: immersing the polyester corrugated conduit obtained in step S4 into a collagen fiber solution of 3.5-5.5mg/mL of the collagen fiber subjected to the first chemical crosslinking so as to perform second chemical crosslinking, standing for 2 hours or more, and drying to obtain a polyester biological patch attached with a collagen fiber coating, wherein the melting point of the collagen fiber coating is 77.59-79.84°C;
Step 3: drying the limiting expandable biological valve to obtain a dried limiting expandable biological valve;
Step 4: assembling the valve holder, the dried limiting expandable biological valve and the aortic valve preliminary conduit to obtain a limiting expandable aortic valve conduit.
